(19)

**Europäisches Patentamt**
**European Patent Office**
**Office européen des brevets**

(11) **EP 4 653 522 A1**

(12) **EUROPEAN PATENT APPLICATION**
published in accordance with Art. 153(4) EPC

(43) Date of publication:
26.11.2025 Bulletin 2025/48

(21) Application number: 24744400.3

(22) Date of filing: 19.01.2024

(51) International Patent Classification (IPC):
*C12N 1/20* (2006.01)        *A61K 35/745* (2015.01)
*A61P 3/04* (2006.01)        *A61P 3/06* (2006.01)
*C12R 1/225* (2006.01)

(52) Cooperative Patent Classification (CPC):
**A61P 29/00; A61K 36/8998; A61P 11/14;**
**A61P 25/04;** A61K 2236/331

(86) International application number:
**PCT/CN2024/073257**

(87) International publication number:
**WO 2024/153231 (25.07.2024 Gazette 2024/30)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: 19.01.2023 US 202363480512 P

(71) Applicant: TCI Co., Ltd
**Taipei 11494 (CN)**

(72) Inventors:
• LIN, Yung-Hsiang
  Taiwan 11494 (TW)
• HUANG, Chu-Han
  Taiwan 11494 (TW)
• CHEN, Wei-Yao
  Taiwan 11494 (TW)

(74) Representative: **Michalski Hüttermann & Partner**
**Patentanwälte mbB**
**Kaistraße 16A**
**40221 Düsseldorf (DE)**

(54) **BIFIDOBACTERIUM BREVE, COMPOSITION COMPRISING BIFIDOBACTERIUM BREVE OR CULTURE THEREOF, AND USE OF BIFIDOBACTERIUM BREVE OR CULTURE THEREOF**

(57) Provided are *Bifidobacterium breve* and a probiotic composition comprising the strain. *Bifidobacterium breve* was deposited in the Bioresource Collection and Research Center of Food Industry Research and Development Institute Taiwan, China on August 8, 2022, and the deposit number BCRC 911145 was obtained. Further provided is use of the strain or a culture thereof in the preparation of a composition for inhibiting maturation of adipocytes, a composition for reducing fat accumulation and/or promoting fat metabolism, a composition for reducing weight, a composition for regulating blood esters, or a composition for regulating blood glucose.

FIG. 1A

**Description**

TECHNICAL FIELD

The present disclosure relates to a probiotic and its application, and in particular it relates to a *Bifidobacterium breve,* a composition containing *Bifidobacterium breve* or a culture thereof, and the use of *Bifidobacterium breve* or a culture thereof.

BACKGROUND TECHNOLOGIES

Overweight, high blood glucose, high blood lipids, etc. are all the main risk factors for cardiovascular disease while healthy diet and moderate exercise are one of the ways to avoid these risk factors.

It is currently known that glucagon-like peptide-1 (GLP-1) belonging to a type of incretin, has the effects of inhibiting gastric motility, delaying gastric emptying, suppressing appetite, promoting insulin secretion from pancreatic islet β-cells, and inhibiting pancreatic glucagon secretion from pancreatic islet α-cells, and it is a hot target for research on appetite regulation, blood sugar modulation, and weight control.

Probiotics are now widely accepted as a health food ingredient. Probiotics are defined as active microorganisms that are present in sufficient amounts to alter the gastrointestinal flora of the host and produce beneficial health effects, wherein bacteria belonging to the genus *Lactobacillus* or belonging to genus *Bifidobacterium* are the most widely used. Probiotics can usually cause a decrease in intestinal pH by lactic acid stimulation, which in turn produces products that contribute to the growth of more beneficial microorganisms and enhance the host's immune response to pathogens.

As mentioned above, a healthy diet is one of the ways to avoid cardiovascular disease risk factors such as overweight, high blood glucose, and high blood lipids, and thus there is still an urgent need to conduct relevant research on probiotics as health foods in appetite regulation, blood glucose modulation, weight control, etc., to achieve effective health management through the consumption of probiotics.

SUMMARY OF THE INVENTION

The present disclosure provides a novel *Bifidobacterium breve* which is deposited in the Bioresource Collection and Research Center of Food Industry Research and Development Institute, and the deposit number of which is BCRC 911145.

The present disclosure also provides a use of the foregoing novel *Bifidobacterium breve* or a culture thereof in the manufacture of a composition for reducing appetite and/or promoting glucagon-like peptide-1 (GLP-1) secretion.

The present disclosure further provides a use of the foregoing novel *Bifidobacterium breve* or a culture thereof in the manufacture of a composition for inhibiting adipocyte maturation.

The present disclosure additionally provides a use of the novel above-mentioned *Bifidobacterium breve* or a culture thereof in the manufacture of a composition for reducing fat accumulation and/or promoting fat metabolism.

Moreover, the present disclosure provides a use of the above-mentioned novel *Bifidobacterium breve* or a culture thereof in the manufacture of a composition for reducing body weight.

The present disclosure further provides a use of the novel *Bifidobacterium breve* mentioned above or a culture thereof in the manufacture of a composition for modulating blood lipids.

The present disclosure provides a use of the novel *Bifidobacterium breve* mentioned above or a culture thereof in the manufacture of a composition for modulating blood glucose.

In addition, the present disclosure further provides a probiotic composition, comprising the aforementioned novel *Bifidobacterium breve* or a culture thereof; and a prebiotic.

In order to make the foregoing and other purposes, features, and advantages of the present disclosure more obvious and easier to understand, the following preferred embodiments, together with the accompanying drawings, are described in detail as follows:

DESCRIPTION OF THE DRAWINGS

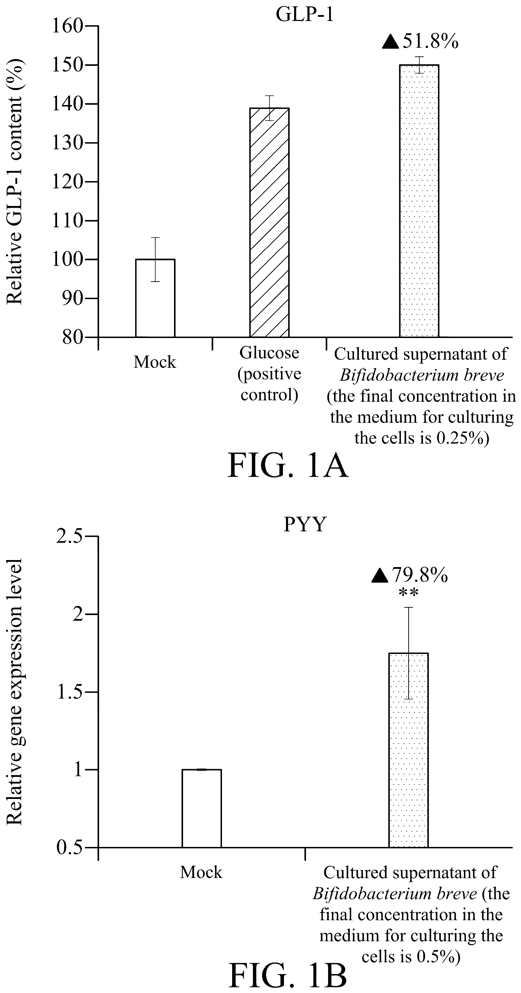

FIG. 1A shows the relative amount of glucagon-like peptide-1 (GLP-1) secreted into the medium by the cells treated with the cultured supernatant of *Bifidobacterium breve* BCRC 911145 (the final concentration of the cultured supernatant of *Bifidobacterium breve* BCRC 911145 in the medium for culturing the cells is 0.25%).

FIG. 1B shows the relative expression of PYY gene in cells treated with the cultured supernatant of *Bifidobacterium breve* BCRC 911145 (cells are cultured for 48 hours, the final concentration of the cultured supernatant of

*Bifidobacterium breve* BCRC 911145 in the medium for culturing the cells is 0.5%). Compared to the Mock group, **: $p < 0.01$.

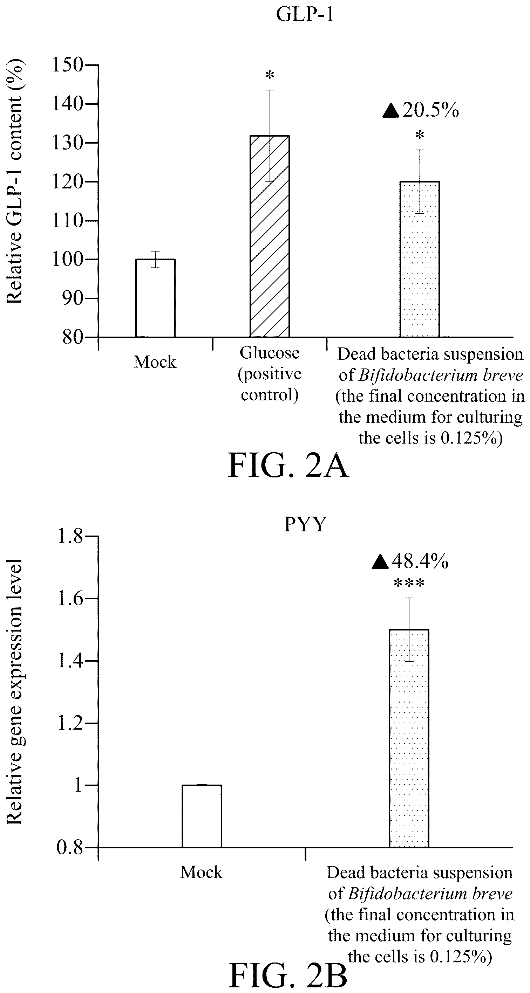

FIG. 2A shows the relative amount of glucagon-like peptide-1 (GLP-1) secreted into the medium by the cells treated with the dead bacteria suspension of *Bifidobacterium breve* BCRC 911145 (the final concentration of the dead bacteria suspension of *Bifidobacterium breve* BCRC 911145 in the medium for culturing the cells is 0.125%). Compared to the Mock group, *: $p < 0.05$.

FIG. 2B shows the relative expression of PYY gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing the cells is 0.125%). Compared to the Mock group, ***: $p < 0.001$.

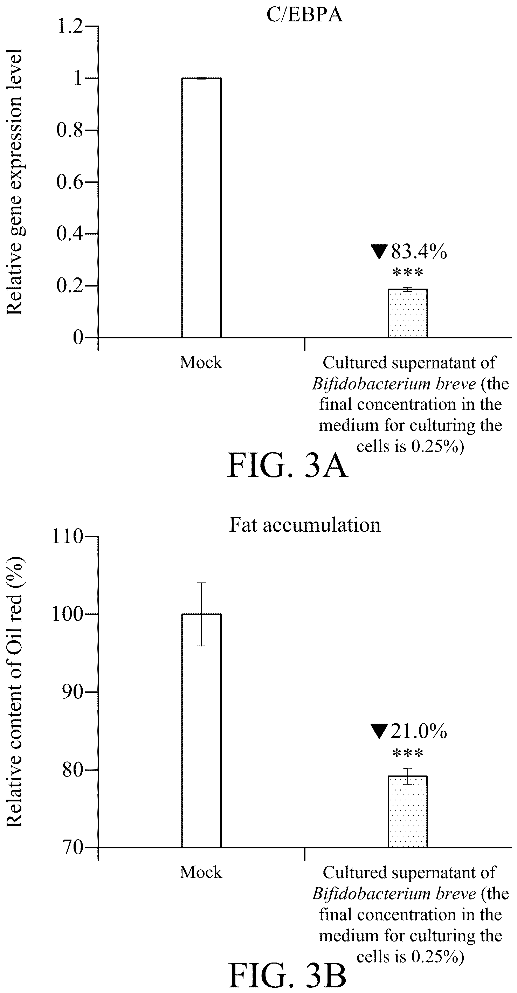

FIG. 3A shows the relative expression of C/EBPA gene in cells treated with the cultured supernatant of *Bifidobacterium breve* BCRC 911145 (cells are cultured for 6 hours, the final concentration of the cultured supernatant of *Bifidobacterium breve* BCRC 911145 in the medium for culturing the cells is 0.25%). Compared to the Mock group, ***: $p < 0.001$.

FIG. 3B shows the Oil red (lipid) content in cells treated with the cultured supernatant of *Bifidobacterium breve* BCRC 911145. Compared to the Mock group, ***: $p < 0.001$.

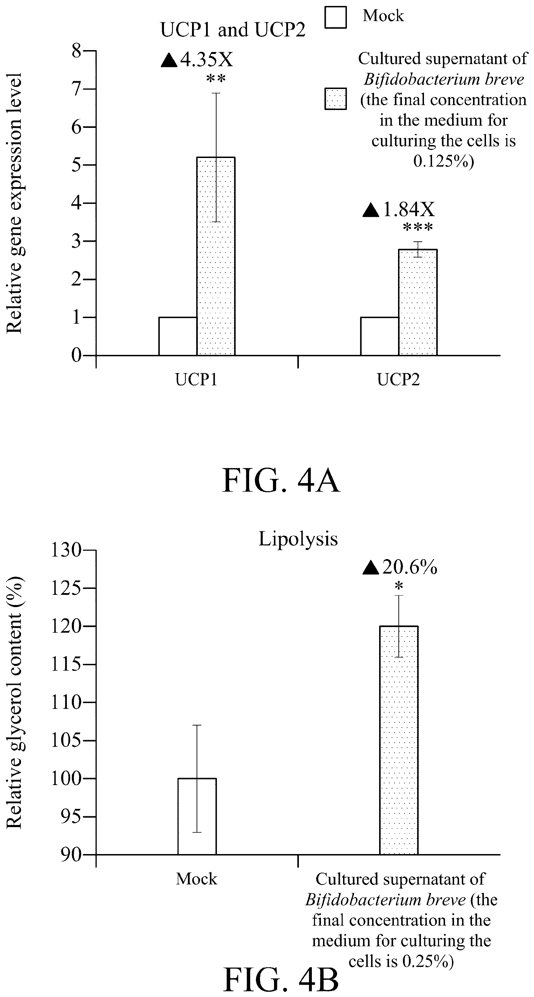

FIG. 4A shows the relative expressions of UCP1 gene and UCP2 gene in cells treated with the cultured supernatant of *Bifidobacterium breve* BCRC 911145 (cells are cultured for 24 hours, the final concentration of the cultured supernatant of *Bifidobacterium breve* BCRC 911145 in the medium for culturing the cells is 0.125%). Compared to the Mock group, **: $p < 0.01$, ***: $p < 0.001$.

FIG. 4B shows the amount of glycerol in the cultured medium of cells treated with the cultured supernatant of *Bifidobacterium breve* BCRC 911145 (the final concentration of the cultured supernatant of *Bifidobacterium breve* BCRC 911145 in the medium for culturing the cells is 0.25%). Compared to the Mock group, *: $p < 0.05$.

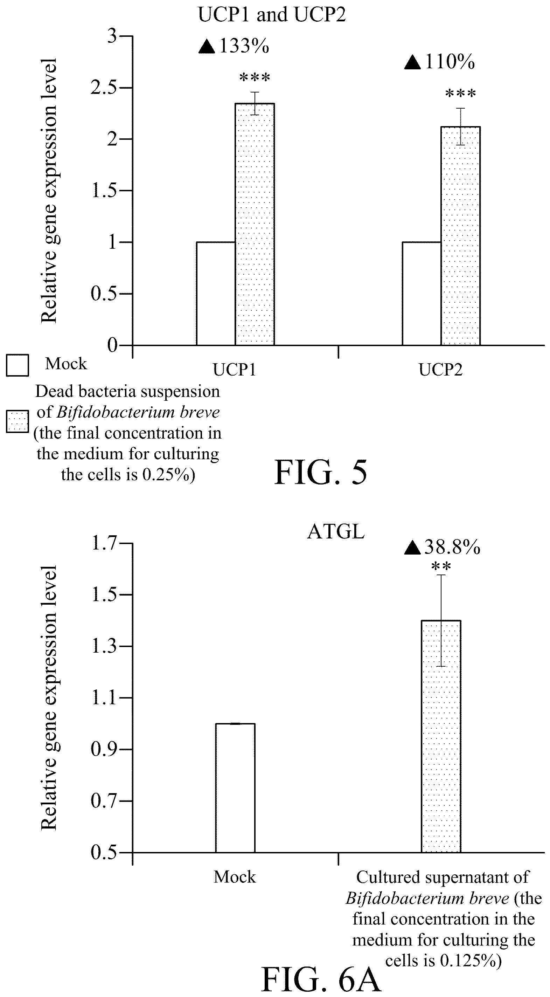

FIG. 5 show the relative expressions of UCP1 gene and UCP2 gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing the cells is 0.25%). Compared to the Mock group, ***: $p < 0.001$.

FIG. 6A shows the relative expression of ATGL gene in cells treated with the cultured supernatant of *Bifidobacterium breve* BCRC 911145 (cells are cultured for 48 hours, the final concentration of the cultured supernatant of *Bifidobacterium breve* BCRC 911145 in the medium for culturing the cells is 0.125%). Compared to the Mock group, **: $p < 0.01$.

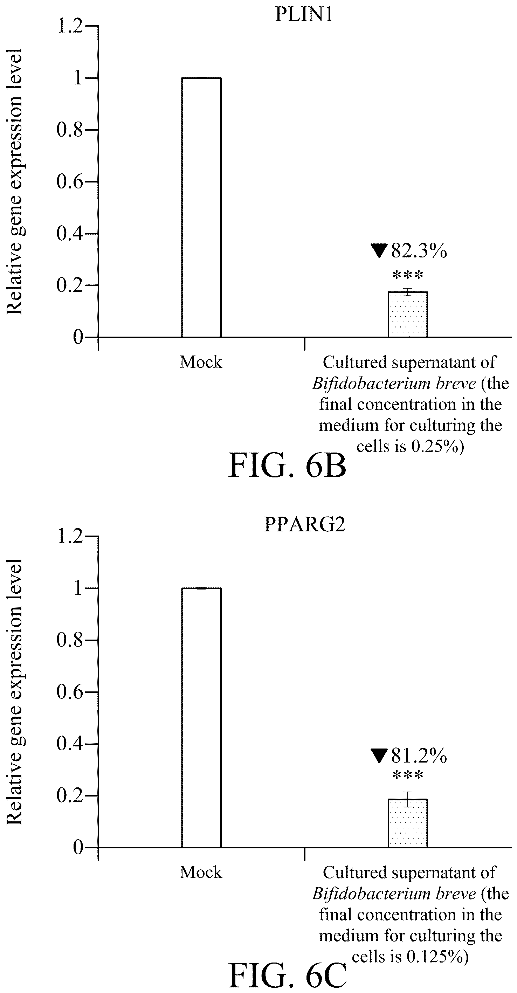

FIG. 6B shows the relative expression of PLIN1 gene in cells treated with the cultured supernatant of *Bifidobacterium breve* BCRC 911145 (cells are cultured for 6 hours, the final concentration of the cultured supernatant of *Bifidobacterium breve* BCRC 911145 in the medium for culturing the cells is 0.25%). Compared to the Mock group, ***: $p < 0.001$.

FIG. 6C shows the relative expression of PPARG2 gene in cells treated with the cultured supernatant of *Bifidobacterium breve* BCRC 911145 (cells are cultured for 24 hours, the final concentration of the cultured supernatant of *Bifidobacterium breve* BCRC 911145 in the medium for culturing the cells is 0.125%). Compared to the Mock group, ***: $p < 0.001$.

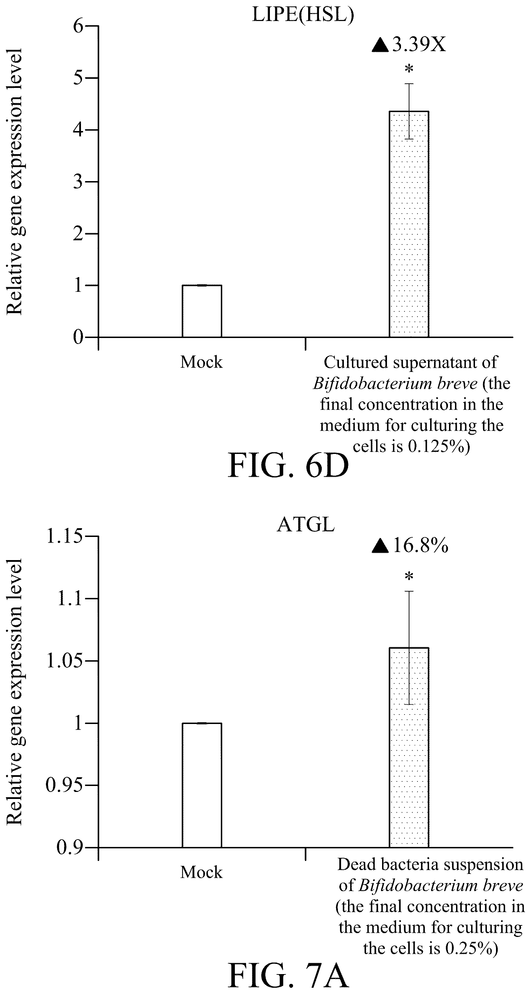

FIG. 6D shows the relative expression of LIPE (HSL) gene in cells treated with the cultured supernatant of *Bifidobacterium breve* BCRC 911145 (cells are cultured for 48 hours, the final concentration of the cultured supernatant of *Bifidobacterium breve* BCRC 911145 in the medium for culturing the cells is 0.125%). Compared to the Mock group, *: $p < 0.05$.

FIG. 7A shows the relative expression of ATGL gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing the cells is 0.25%). Compared to the Mock group, *: $p < 0.05$.

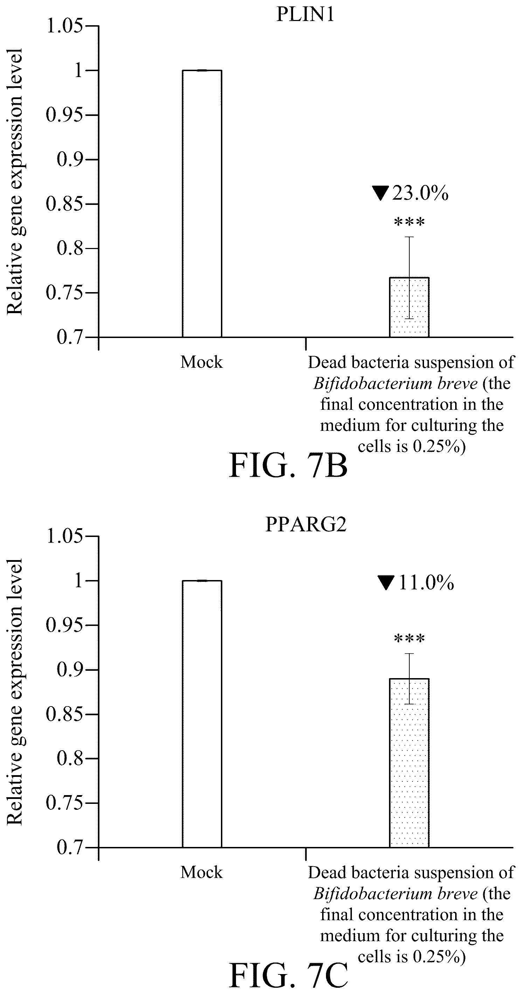

FIG. 7B shows the relative expression of PLIN1 gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing the cells is 0.25%). Compared to the Mock group, ***: $p < 0.001$.

FIG. 7C shows the relative expression of PPARG2 gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing the cells is 0.25%). Compared to the Mock group, ***: $p < 0.001$.

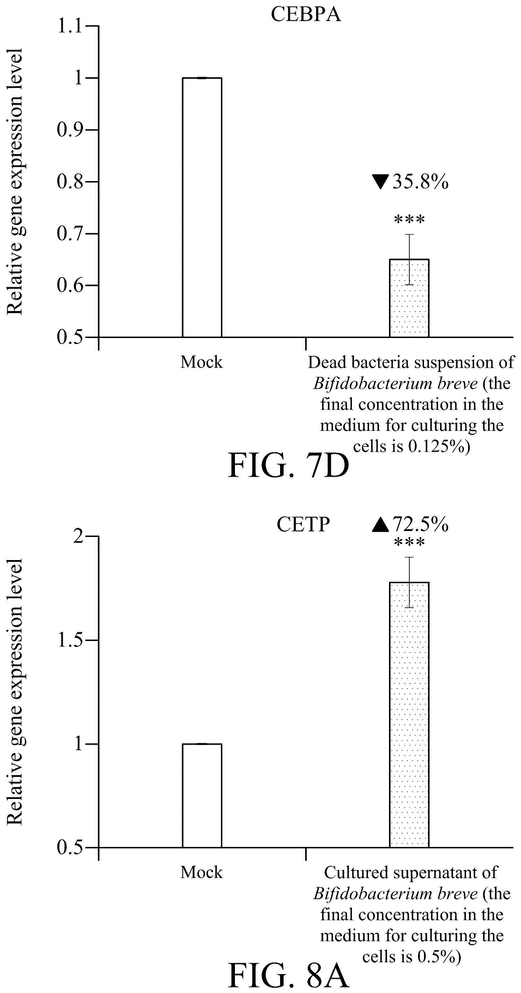

FIG. 7D shows the relative expression of CEBPA gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing the cells is 0.125%). Compared to the Mock group, ***: $p < 0.001$.

FIG. 8A shows the relative expression of CETP gene in cells treated with the cultured supernatant of *Bifidobacterium breve* BCRC 911145 (cells are cultured for 6 hours, the final concentration of the cultured supernatant of *Bifidobacterium breve* BCRC 911145 in the medium for culturing the cells is 0.5%). Compared to the Mock group, ***: $p < 0.001$.

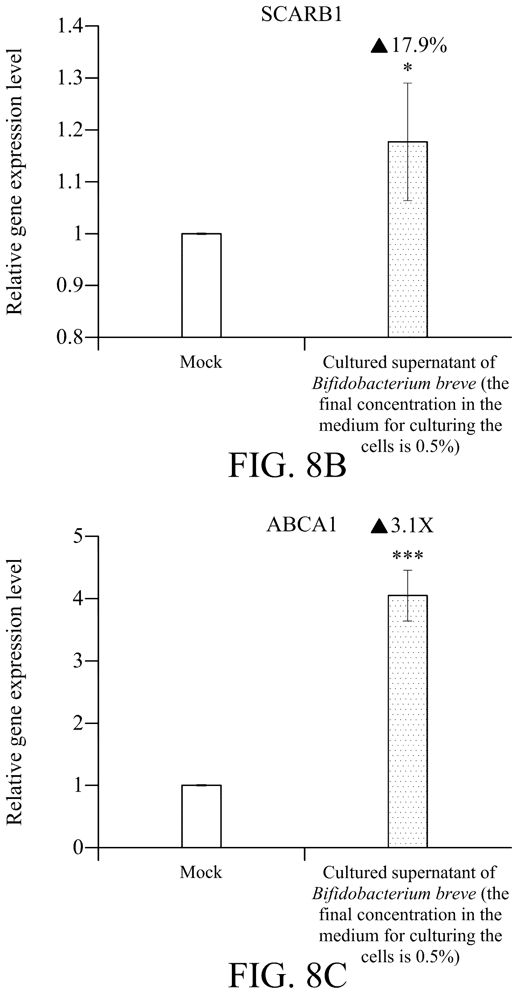

FIG. 8B shows the relative expression of SCARB1 gene in cells treated with the cultured supernatant of *Bifidobacterium breve* BCRC 911145 (cells are cultured for 6 hours, the final concentration of the cultured supernatant of *Bifidobacterium breve* BCRC 911145 in the medium for culturing the cells is 0.5%). Compared to the Mock group, *: $p < 0.05$.

FIG. 8C shows the relative expression of ABCA1 gene in cells treated with the cultured supernatant of *Bifidobacterium breve* BCRC 911145 (cells are cultured for 6 hours, the final concentration of the cultured supernatant of *Bifidobacterium breve* BCRC 911145 in the medium for culturing the cells is 0.5%). Compared to the Mock group, ***: $p < 0.001$.

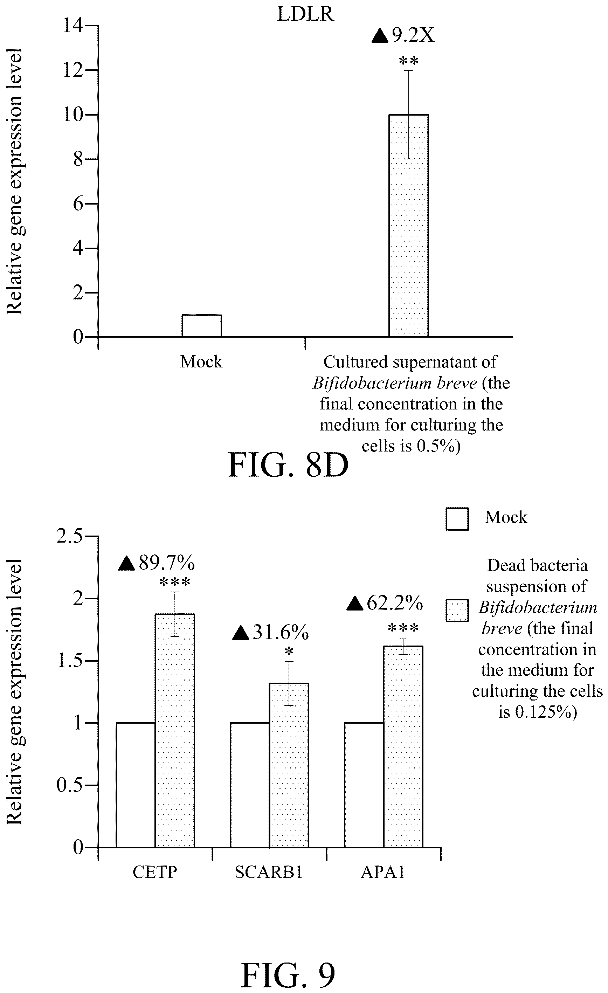

FIG. 8D shows the relative expression of LDLR gene in cells treated with the cultured supernatant of *Bifidobacterium breve* BCRC 911145 (cells are cultured for 48 hours, the final concentration of the cultured supernatant of *Bifidobacterium breve* BCRC 911145 in the medium for culturing the cells is 0.5%). Compared to the Mock group, **: $p < 0.01$.

FIG. 9 show the relative expressions of CETP gene, SCARB1 gene and ABCA1 gene in cells treated with the dead bacteria suspension of *Bifidobacterium breve* BCRC 911145 (cells are cultured for 6 hours, the final concentration of the dead bacteria suspension of *Bifidobacterium breve* BCRC 911145 in the medium for culturing the cells is 0.125%). Compared to the Mock group, *: $p < 0.05$, **: $p < 0.01$, ***: $p < 0.001$.

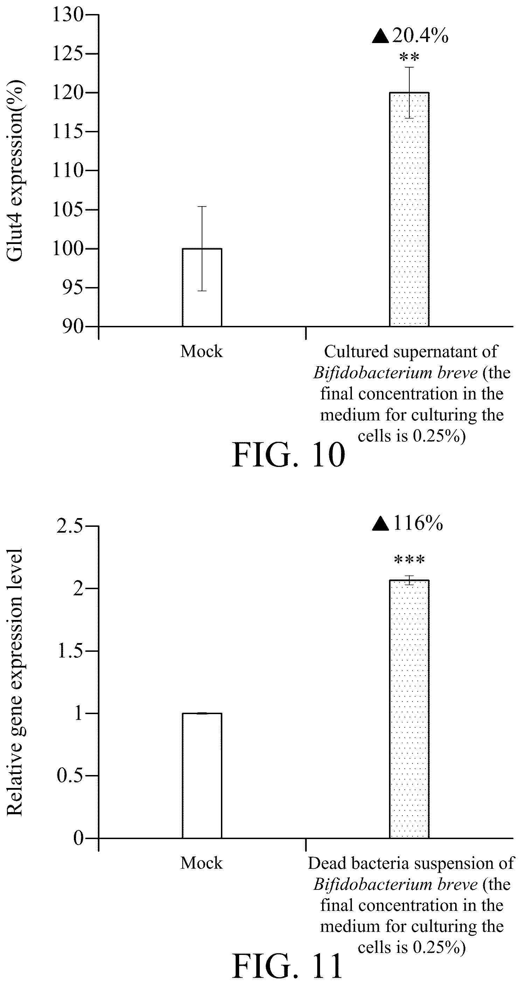

FIG. 10 show the expression of glucose transporter type 4 (GLUT4) in cells treated with the cultured supernatant of *Bifidobacterium breve* BCRC 911145. Compared to the Mock group, **: $p < 0.01$.

FIG. 11 show the relative expression of glucose transporter type 4 (GLUT4) in cells treated with the dead bacteria suspension of *Bifidobacterium breve* BCRC 911145. Compared to the Mock group, ***: $p < 0.001$.

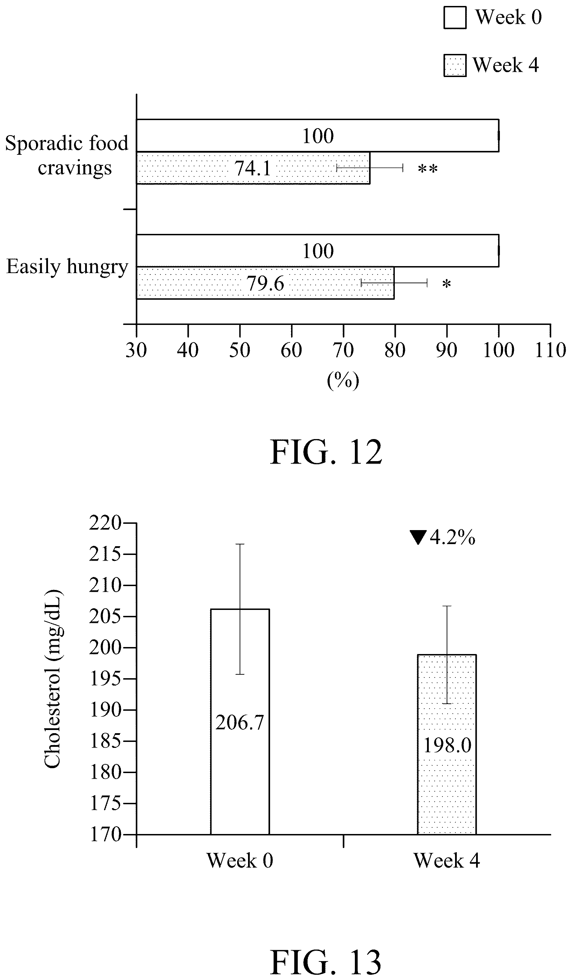

FIG. 12 shows the results of the survey on the appetite status of the subjects in the experiment for efficacy test of viable bacteria powder. Compared to Week 0, *: $p < 0.05$, **: $p < 0.01$.

FIG. 13 shows the changes in total cholesterol of the subjects in the experiment for efficacy test of viable bacteria powder. Compared to Week 0, *: $p < 0.05$. Total cholesterol reference value: <200 mg/dL.

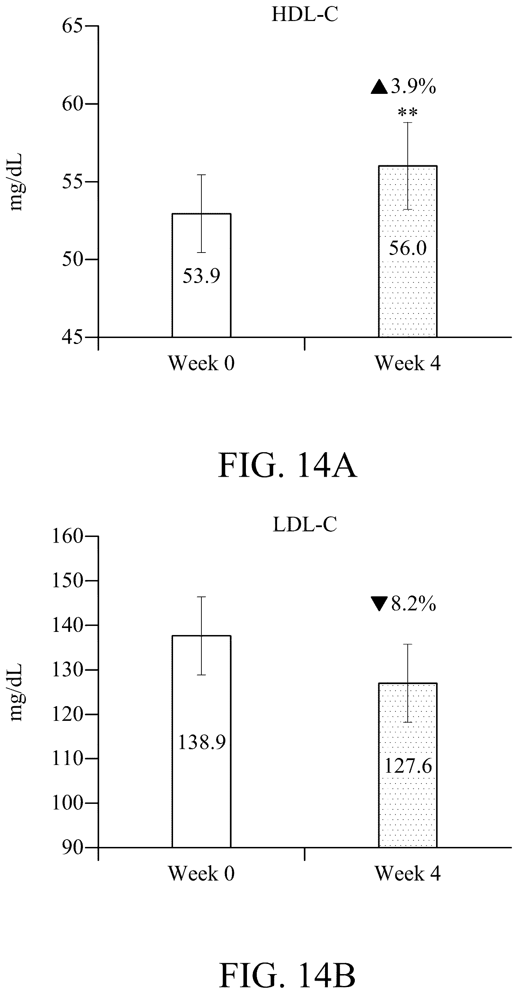

FIG. 14A shows the changes in high-density lipoprotein cholesterol (HDL-C) of the subjects in the experiment for efficacy test of viable bacteria powder. Compared to Week 0, **: $p < 0.01$. High-density lipoprotein cholesterol (HDL-C) reference value: >40 mg/dL.

FIG. 14B shows the changes in low-density lipoprotein cholesterol (LDL-C) of the subjects in the experiment for efficacy test of viable bacteria powder. Low-density lipoprotein cholesterol (LDL-C) reference value: <100 mg/dL.

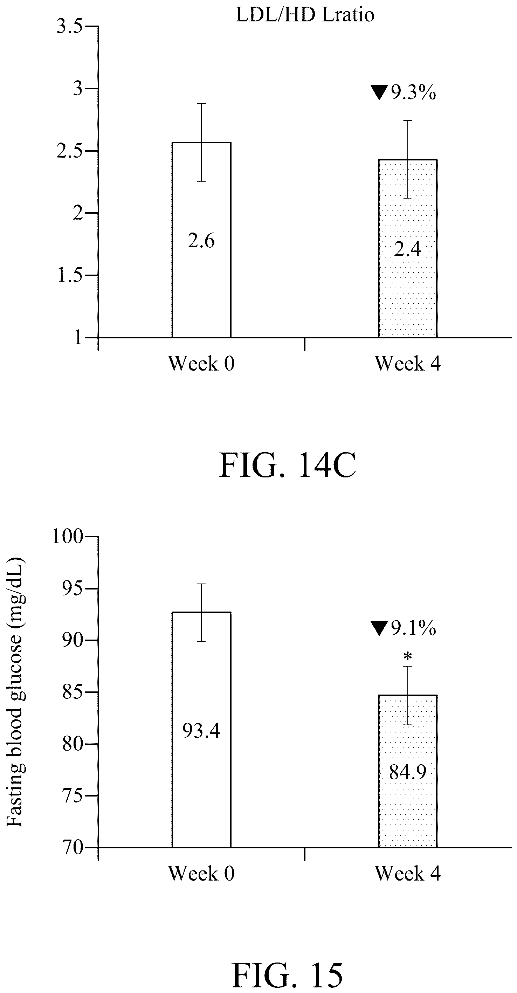

FIG. 14C shows the changes in the ratio of low-density lipoprotein cholesterol to high-density lipoprotein cholesterol (LDL/HDL) of the subjects in the experiment for efficacy test of viable bacteria powder. Reference values for the ratio of low-density lipoprotein cholesterol to high-density lipoprotein cholesterol: Male: <3.55; Female: <3.22.

FIG. 15 shows the changes in fasting blood glucose of the subjects in the experiment for efficacy test of viable bacteria powder. Compared to Week 0, *: $p < 0.05$. Fasting blood glucose reference value: 74-109 mg/dL.

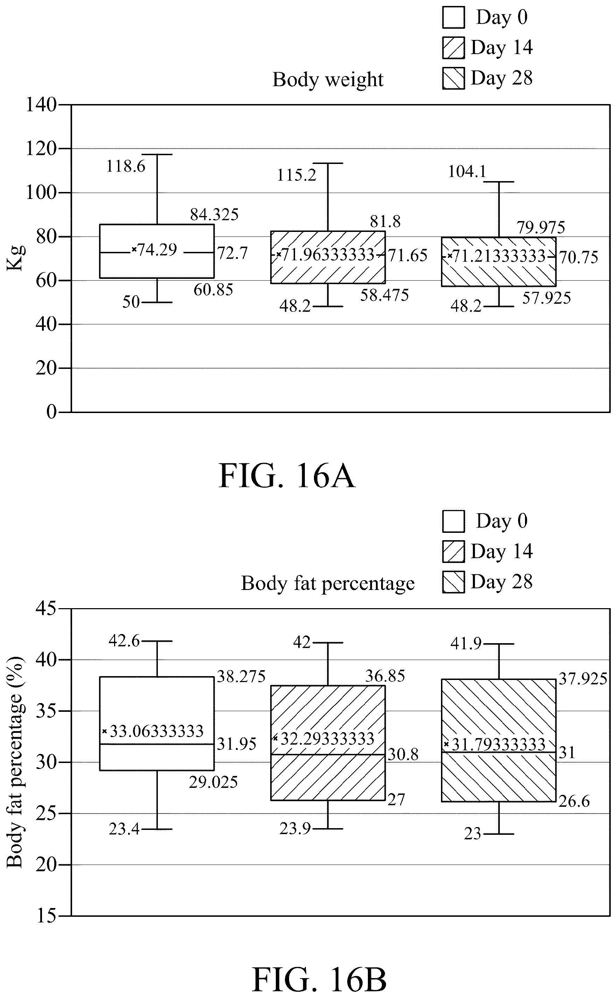

FIG. 16A shows the changes in body weight of subjects in the experiment for efficacy test of composition containing viable bacteria and soybean extract.

FIG. 16B shows the changes in body fat percentage of subjects in the experiment for efficacy test of composition containing viable bacteria and soybean extract.

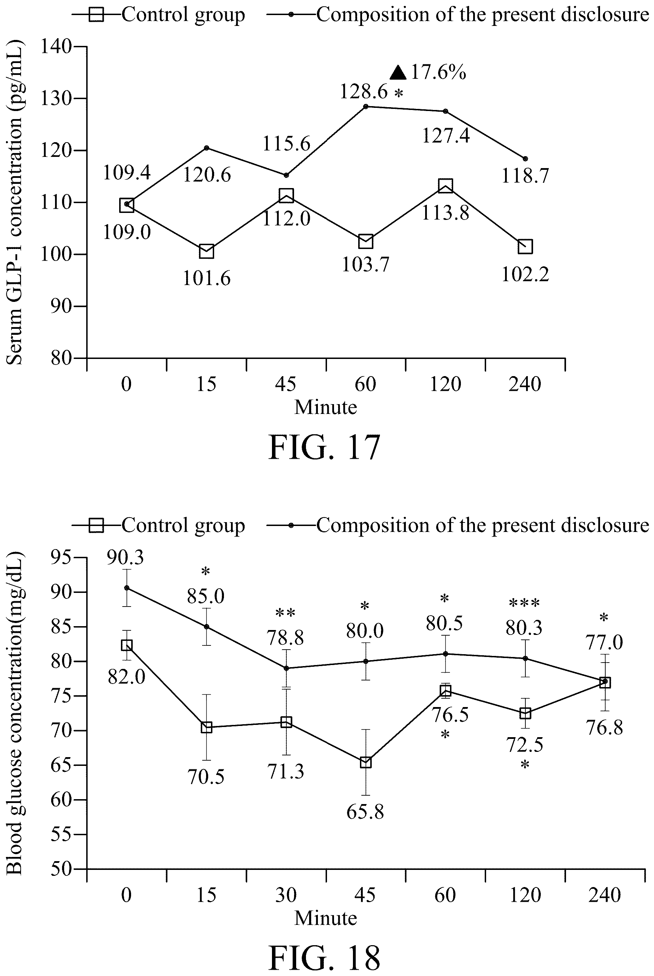

FIG. 17 shows the changes in serum glucagon-like peptide-1 (GLP-1) concentrations of subjects in the experiment for blood glucose modulating efficacy test of composition containing viable bacteria, soybean extract and apple extract (Mode 1). For the same group of subjects, blood samples are collected at the same time intervals when the subjects do not take the composition of the present disclosure (the control group) and when the subjects take the composition of the present disclosure (the group taking the composition of the present disclosure). Compared to the baseline (fasting blood test value before the subject takes the composition of the present disclosure (not taking the composition of the present disclosure) (0 hour)), *: $p < 0.05$.

FIG. 18 shows the changes in blood glucose concentrations of subjects in the experiment for blood glucose modulating efficacy test of composition containing viable bacteria, soybean extract and apple extract (Mode 1). For the same group of subjects, blood samples are collected at the same time intervals when the subjects do not take the composition of the present disclosure (the control group) and when the subjects take the composition of the present disclosure (the group taking the composition of the present disclosure). Compared to the baseline (fasting blood test value before the subject takes the composition of the present disclosure (not taking the composition of the present disclosure) (0 hour)), *: $p < 0.05$, **: $p < 0.01$, ***: $p < 0.001$.

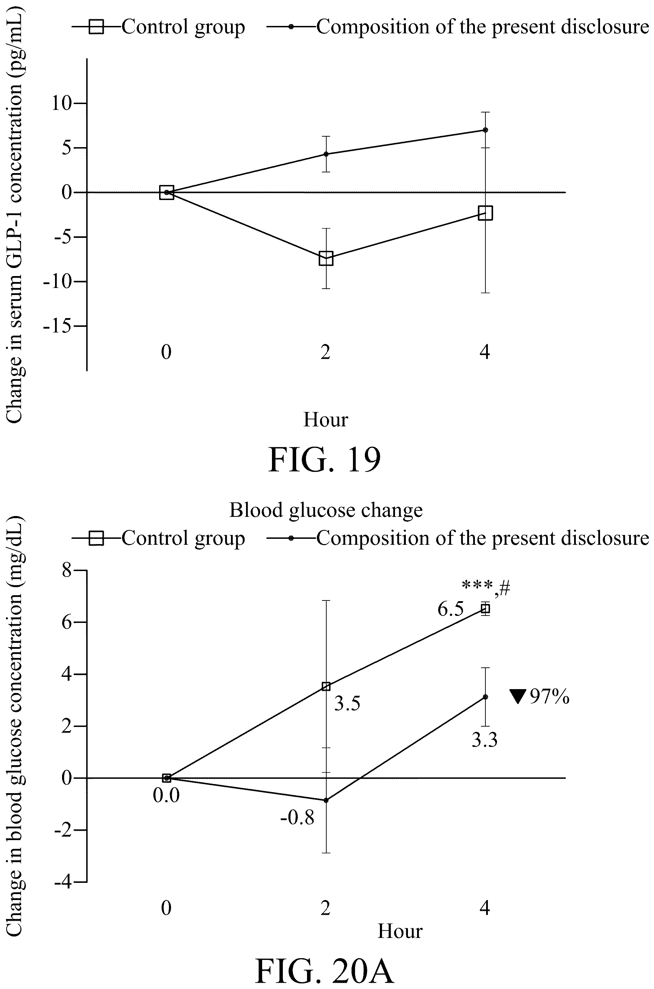

FIG. 19 shows the changes in serum glucagon-like peptide-1 (GLP-1) concentrations of subjects in the experiment for blood glucose modulating efficacy test of composition containing viable bacteria, soybean extract and apple extract (Mode 2). For the same group of subjects, blood samples are collected at the same time intervals when the subjects do not take the composition of the present disclosure (the control group) and when the subjects take the composition of the present disclosure (the group taking the composition of the present disclosure).

FIG. 20A shows the changes in blood glucose concentrations of subjects in the experiment for blood glucose modulating efficacy test of composition containing viable bacteria, soybean extract and apple extract (Mode 2). For the same group of subjects, blood samples are collected at the same time intervals when the subjects do not take the composition of the present disclosure (the control group) and when the subjects take the composition of the present disclosure (the group taking the composition of the present disclosure). Compared to the baseline (fasting blood test value before the subject takes the composition of the present disclosure (not taking the composition of the present disclosure) (0 hour)), ***: $p < 0.001$. Compared to the control group, #: $p < 0.05$.

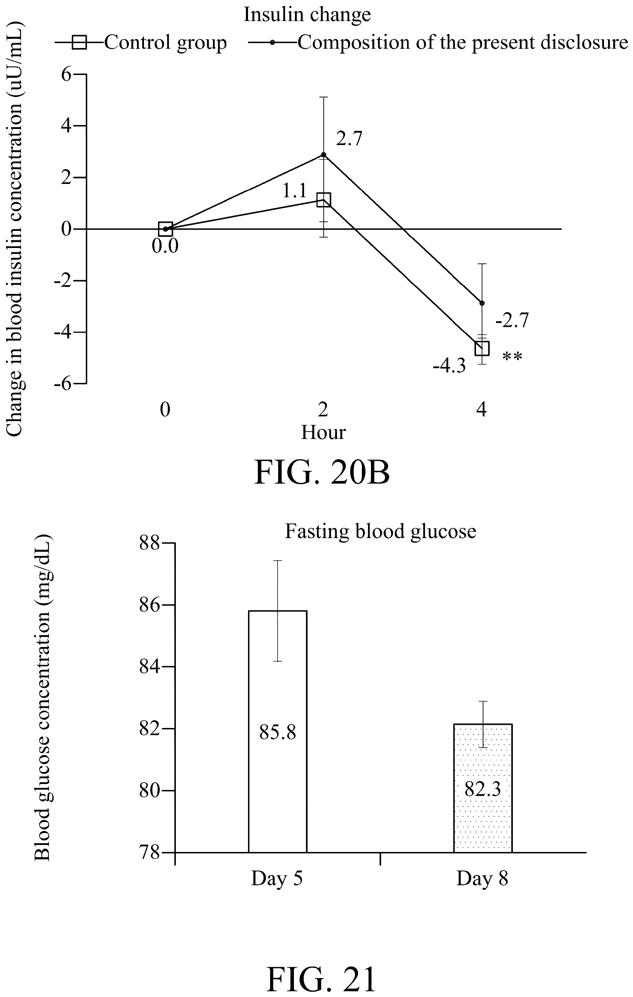

FIG. 20B shows the changes in blood insulin concentrations of subjects in the experiment for blood glucose modulating efficacy test of composition containing viable bacteria, soybean extract and apple extract (Mode 2). For the same group of subjects, blood samples are collected at the same time intervals when the subjects do not take the composition of the present disclosure (the control group) and when the subjects take the composition of the present disclosure (the group taking the composition of the present disclosure). Compared to the baseline (fasting blood test value before the subject takes the composition of the present disclosure (not taking the composition of the present disclosure) (0 hour)), **: $p < 0.01$.

FIG. 21 shows in the experiment for blood glucose modulating efficacy test of composition containing viable bacteria, soybean extract and apple extract (Mode 2), changes in fasting blood glucose concentrations of subjects on the Day 5 and Day 8 (after taking the above composition for 3 consecutive days (Day 5 to Day 7)) of the experiment.

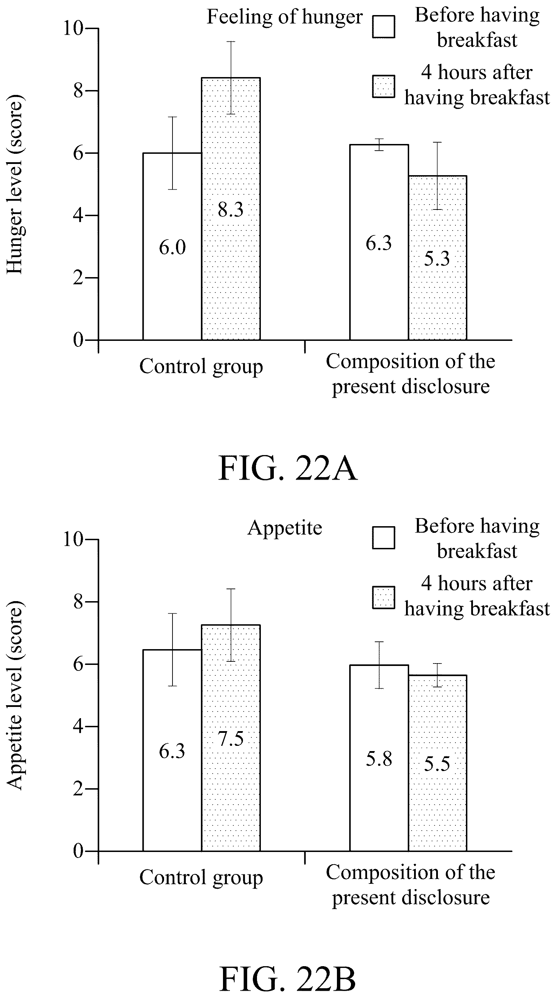

FIG. 22A shows survey results on the subjects' feeling of hunger in the experiment for blood glucose modulating efficacy test of composition containing viable bacteria, soybean extract and apple extract (Mode 2).

FIG. 22B shows survey results on the subjects' appetite level in the experiment for blood glucose modulating efficacy test of composition containing viable bacteria, soybean extract and apple extract (Mode 2).

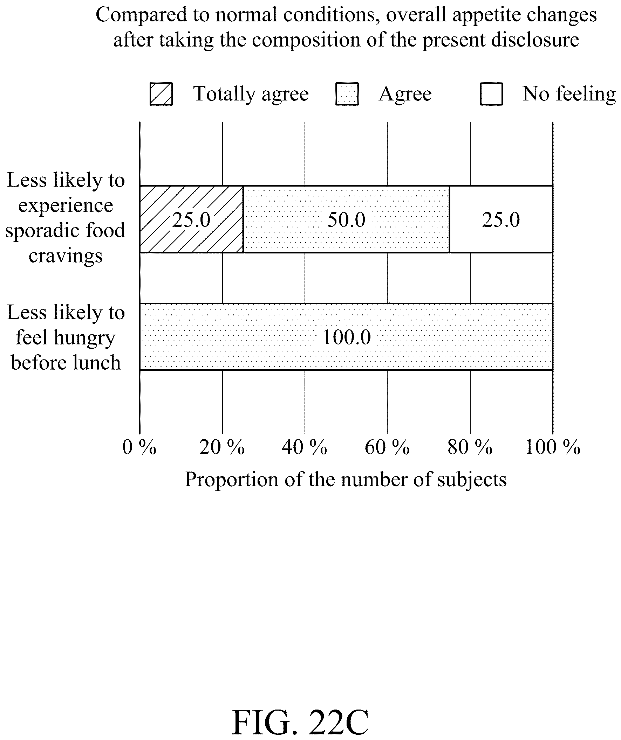

FIG. 22C shows survey results on subjects' overall appetite changes in the experiment for blood glucose modulating efficacy test of composition containing viable bacteria, soybean extract and apple extract (Mode 2).

## DETAILED DESCRIPTION OF THE INVENTION

The present disclosure may provide a probiotic, which may be an isolated probiotic. The foregoing isolated probiotic of the present disclosure may be *Bifidobacterium breve.* In one embodiment, the foregoing isolated probiotic of the present disclosure may be a novel *Bifidobacterium breve* which was deposited in the Bioresource Collection and Research Center (BCRC) of Food Industry Research and Development Institute (FIRDI), Taiwan, China on August 8, 2022, and obtained the deposit number BCRC 911145, and which was also deposited in the German Collection of Microorganisms and Cell Cultures GmbH (DSMZ) on September 7, 2022 and obtained the deposit number DSM 34383 while for the sake of conciseness, this novel *Bifidobacterium breve* hereinafter will be referred to *Bifidobacterium breve* BCRC 911145 in the content of the present disclosure.

Effects of the *Bifidobacterium breve* BCRC 911145 of the present disclosure may comprise, but is not limited to, reducing appetite, promoting glucagon-like peptide-1 (GLP-1) secretion, inhibiting adipocyte maturation, reducing fat accumulation, promoting fat metabolism, reducing body weight, modulating blood lipids (such as reducing total cholesterol, promoting high-density lipoprotein cholesterol (HDL-C) synthesis, promoting low-density lipoprotein cholesterol (LDL-C) metabolism), modulating blood glucose, etc., or any combination of the above.

There is no particular limitation on the application of the *Bifidobacterium breve* BCRC 911145 of the present disclosure, for example, the *Bifidobacterium breve* BCRC 911145 of the present disclosure may be applied to the fields related to health management and/or medical treatment etc., especially may be applied to the areas of appetite control, body weight control, body fat modulation, blood lipid modulation, blood glucose modulation, etc., but it is also not limited thereto.

In one embodiment, the aforementioned *Bifidobacterium breve* BCRC 911145 of the present disclosure may be prepared as an oral dosage form. Examples of the above-mentioned oral dosage form may include, but are not limited to, a powder, a granule, a tablet, a troche, a pill, a capsule, a lozenge, a solution, a suspension, an emulsion, a syrup, an elixir or a slurry, etc.

In one specific embodiment, the aforementioned *Bifidobacterium breve* BCRC 911145 of the present disclosure may be prepared as a powder for oral dosage form. In another specific embodiment, the aforementioned *Bifidobacterium breve* BCRC 911145 of the present disclosure may be prepared as a capsule for oral dosage form. In yet another specific embodiment, the aforementioned *Bifidobacterium breve* BCRC 911145 of the present disclosure may be prepared as a solution for oral dosage form.

The present disclosure may also provide a probiotic culture which is a culture of any foregoing probiotic of the

**EP 4 653 522 A1**

present disclosure while the probiotic described herein may include *Bifidobacterium breve.* In one embodiment, the probiotic described herein may be foregoing *Bifidobacterium breve* BCRC 911145.

The probiotic culture of the present disclosure mentioned above may comprise the aforementioned probiotic which has been cultured (i.e., the probiotic which has been cultured alone), a cultured medium containing the aforementioned probiotic which has been cultured (i.e., the probiotic which has been cultured plus the cultured medium), or a cultured supernatant in which the aforementioned probiotic which has been cultured has been removed (i.e., the cultured medium alone), but it is not limited thereto.

The manner of separating the foregoing probiotic which has been cultured from the cultured medium to obtain the individual foregoing probiotic which has been cultured and foregoing cultured supernatant (cultured medium alone) is not particularly limited, as long as the two can be separated. For example, the foregoing probiotic which has been cultured may be separated from the cultured medium by means such as centrifugation, filtration, or the like, but it is not limited thereto.

Moreover, short-chain fatty acid (SCFA) contained in the cultured medium or cultured supernatant in the probiotic culture of the present disclosure may include at least one of the following, but is not limited to: formic acid, acetic acid, propionic acid, butyric acid, isobutyric acid, 3-methylbutyric acid, etc.

For the probiotic culture of the present disclosure mentioned above, in one embodiment, the aforementioned probiotic which has been cultured may be active or maintain its activity, or may be a viable bacterium.

Furthermore, for the probiotic culture of the present disclosure mentioned above, in another embodiment, the aforementioned probiotic which has been cultured may have been stripped of its own physiological activity (the bacterium loses its physiological functions), or may be a dead bacterium. There is no particular limitation on a manner of making the aforementioned probiotic which has been cultured be stripped of its own physiological activity or became a dead bacterium, as long as it can make the aforementioned probiotic which has been cultured be stripped of its physiological activity or became a dead bacterium. In one embodiment, the probiotic which has been cultured mentioned above can be made be stripped of its physiological activity or became a dead bacterium by a physical mean. For example, the probiotic which has been cultured mentioned above can be made be stripped of its physiological activity (the bacterium loses its physiological functions) or became a dead bacterium by high temperature and/or high pressure, but it is not limited thereto.

In one embodiment, the probiotic culture of the present disclosure mentioned above may be the foregoing probiotic which has been cultured (i.e., the probiotic which has been cultured alone). For this embodiment, in one specific embodiment, the foregoing probiotic which has been cultured may be active or maintain its activity, or may be a viable bacterium, however, in another specific embodiment, the foregoing probiotic which has been cultured may have been stripped of its own physiological activity (the bacterium loses its physiological functions), or may be a dead bacterium.

In another embodiment, the probiotic culture of the present disclosure mentioned above may be a cultured medium containing the aforementioned probiotic which has been cultured (i.e., the probiotic which has been cultured plus the cultured medium). For this embodiment, in one specific embodiment, the foregoing probiotic which has been cultured may be active or maintain its activity, or may be a viable bacterium, however, in another specific embodiment, the foregoing probiotic which has been cultured may have been stripped of its own physiological activity (the bacterium loses its physiological functions), or may be a dead bacterium.

In further another embodiment, the probiotic culture of the present disclosure mentioned above may be a cultured supernatant in which the aforementioned probiotic have been removed (i.e., the cultured medium alone). For this embodiment, in one specific embodiment, the foregoing cultured supernatant may be sterilized and/or disinfected, however, in another specific embodiment, the foregoing cultured supernatant may be non-sterilized and/or non-disinfected. There is no particular limitation on a manner for sterilizing and/or disinfecting the cultured supernatant, as long as it can make the cultured supernatant be sterilized and/or stripped of toxins that may be contained therein. For example, the cultured supernatant can be made be sterilized and/or stripped of toxins that may be contained therein by high temperature and/or high pressure, but it is not limited thereto.

Furthermore, for the probiotic culture of the present disclosure mentioned above, there is also no particular limitation on a medium used to culture the aforementioned probiotic to produce the probiotic culture of the present disclosure mentioned above, as long as it can make the aforementioned probiotic grow. Examples of a medium that can be used to culture the aforementioned probiotic to produce the probiotic culture of the present disclosure mentioned above may include, but is not limited to, MRS broth (DeMan-Rogosa-Sharpe broth), Lysogeny broth (LB), skim milk medium, etc. For the probiotic culture of the present disclosure mentioned above, in one embodiment, a medium used to culture the foregoing probiotic to produce the probiotic culture of the present disclosure mentioned above may be MRS broth.

In one embodiment, the probiotic culture of the present disclosure mentioned above may be prepared as an oral dosage form. Examples of the above-mentioned oral dosage form may include, but are not limited to, a powder, a granule, a tablet, a troche, a pill, a capsule, a lozenge, a solution, a suspension, an emulsion, a syrup, an elixir or a slurry, etc.

In one specific embodiment, the aforementioned probiotic culture of the present disclosure may be prepared as a powder for oral dosage form. In another specific embodiment, the aforementioned probiotic culture of the present disclosure may be prepared as a capsule for oral dosage form. In yet another specific embodiment, the aforementioned probiotic culture of the present disclosure may be prepared as a solution for oral dosage form.

In addition, for any of the probiotic culture of the present disclosure mentioned above, in one specific embodiment, said probiotic may be the aforementioned *Bifidobacterium breve* BCRC 911145.

The probiotic culture of the present disclosure mentioned above may have effects of reducing appetite, promoting glucagon-like peptide-1 (GLP-1) secretion, inhibiting adipocyte maturation, reducing fat accumulation, promoting fat metabolism, reducing body weight, modulating blood lipids (such as reducing total cholesterol, promoting high-density lipoprotein cholesterol (HDL-C) synthesis, promoting low-density lipoprotein cholesterol (LDL-C) metabolism), modulating blood glucose, etc., or any combination of the above, but it is not limited thereto.

Moreover, there is also no particular limitation on the application of the probiotic culture of the present disclosure mentioned above. For example, the probiotic culture of the present disclosure mentioned above may be applied to the fields related to health management and/or medical treatment etc., especially may be applied to the areas of appetite control, body weight control, body fat modulation, blood lipid modulation, blood glucose modulation, etc., but it is not limited thereto.

Based on the foregoing, the present disclosure may also provide the following uses of any of the aforementioned probiotic of the present disclosure or any of the aforementioned culture thereof, but it is not limited thereto:

(i) A use of any of the aforementioned probiotic of the present disclosure or any of the aforementioned culture thereof in the manufacture of a composition for reducing appetite and/or promoting glucagon-like peptide-1 (GLP-1) secretion;
(ii) A use of any of the aforementioned probiotic of the present disclosure or any of the aforementioned culture thereof in the manufacture of a composition for inhibiting adipocyte maturation;
(iii) A use of any of the aforementioned probiotic of the present disclosure or any of the aforementioned culture thereof in the manufacture of a composition for reducing fat accumulation and/or promoting fat metabolism;
(iv) A use of the aforementioned probiotic of the present disclosure or any of the aforementioned culture thereof in the manufacture of a composition for reducing body weight;
(v) A use of the aforementioned probiotic of the present disclosure or any of the aforementioned culture thereof in the manufacture of a composition for modulating blood lipids. Said modulating blood lipids may comprise reducing total cholesterol, promoting high-density lipoprotein cholesterol (HDL-C) synthesis, promoting low-density lipoprotein cholesterol (LDL-C) metabolism, etc., but it is not limited thereto; and/or
(vi) A use of the aforementioned probiotic of the present disclosure or any of the aforementioned culture thereof in the manufacture of a composition for modulating blood glucose.

Furthermore, based on the foregoing, the present disclosure may further provide the following method, but it is not limited thereto:

(i) A method for reducing appetite and/or promoting glucagon-like peptide-1 (GLP-1) secretion, comprising, but not limited to, administering any of the probiotic of the present disclosure mentioned above or any of the culture thereof mentioned above to a subject in need thereof;
(ii) A method for inhibiting adipocyte maturation, comprising, but not limited to, administering any of the probiotic of the present disclosure mentioned above or any of the culture thereof mentioned above to a subject in need thereof;
(iii) A method for reducing fat accumulation and/or promoting fat metabolism, comprising, but not limited to, administering any of the probiotic of the present disclosure mentioned above or any of the culture thereof mentioned above to a subject in need thereof;
(iv) A method for reducing body weight, comprising, but not limited to, administering any of the probiotic of the present disclosure mentioned above or any of the culture thereof mentioned above to a subject in need thereof;
(v) A method for modulating blood lipids, comprising, but not limited to, administering any of the probiotic of the present disclosure mentioned above or any of the culture thereof mentioned above to a subject in need thereof. The modulating blood lipids may comprise reducing total cholesterol, promoting high-density lipoprotein cholesterol (HDL-C) synthesis, promoting low-density lipoprotein cholesterol (LDL-C) metabolism, etc., but it is not limited thereto; and/or
(vi) A method for modulating blood glucose, comprising, but not limited to, administering any of the probiotic of the present disclosure mentioned above or any of the culture thereof mentioned above to a subject in need thereof.

The subject mentioned above may comprise a vertebrate, but it is not limited thereto. Moreover, the vertebrate mentioned above may include a fish, an amphibian, a reptile, a bird, or a mammal, but it is not limited thereto. Examples of the mammal may include, but are not limited to, a human, an orangutan, a monkey, a horse, a donkey, a llama, a goat, a pig, a dog, a cat, a rabbit, a guinea pig, a rat, and a mouse. In one embodiment, the subject mentioned above may be a human.

Moreover, the present disclosure may further provide a probiotic composition. The foregoing probiotic composition of the present disclosure may comprise, but is not limited to, any of the probiotic of the present disclosure mentioned above or any of the culture thereof mentioned above and a prebiotic.

In the probiotic composition of the present disclosure, the weight ratio of the foregoing probiotic of the present disclosure or the culture thereof to the foregoing prebiotic may be about 1-6:1-12, such as about 1-5.5:1.5-11, about 1-5:1.5-10, about 1-4:2-9, about 1-3:3-5, about 1:1, about 1:1.5, about 1:2, about 1:2.5, about 1:3, about 1.5:1, about 2:1, about 2:1.5, about 2:3, about 3:1, about 3:8, about 4:7, about 5:6, about 6:5, about 6:7, but it is not limited thereto. In one embodiment, in the probiotic composition of the present disclosure, the weight ratio of the foregoing probiotic of the present disclosure or the culture thereof to the foregoing prebiotic may be about 1:1. In another embodiment, in the probiotic composition of the present disclosure, the weight ratio of the foregoing probiotic of the present disclosure or the culture thereof to the foregoing prebiotic may be about 1:1.5. In yet another embodiment, in the probiotic composition of the present disclosure, the weight ratio of the foregoing probiotic of the present disclosure or the culture thereof to the foregoing prebiotic may be about 1:2.

The foregoing prebiotic may comprise at least one of the following ingredients, but it is not limited thereto: a soybean extract, an oat extract, a barley extract, an apple extract, a banana extract, a tomato extract, a chicory extract, an asparagus extract, a beet extract, a garlic extract, an onion extract, a seaweed extract, a pectin extract, a tea extract, etc.

In one embodiment, in the probiotic composition of the present disclosure, the foregoing prebiotic may be a soybean extract. In this embodiment, the weight ratio of the foregoing probiotic of the present disclosure or the culture thereof to the foregoing soybean extract may be about 1-6:1-6, such as about 1-5:1-5, about 1.5-4:1.5-4, about 2-5:2-5, about 2.5-6:2.5-6, about 1:1, about 1:1.5, about 1:2, about 1:2.5, about 1:3, about 1:4, about 1.5:1, about 2:1, about 2.5:1, about 3:1, about 4:1, about 2:3, about 3:2, about 5:6, about 6:5, but it is not limited thereto. Moreover, in one specific embodiment of this embodiment, the weight ratio of the foregoing probiotic of the present disclosure or the culture thereof to the foregoing soybean extract may be about 1:1. In another specific embodiment of this embodiment, the weight ratio of the foregoing probiotic of the present disclosure or the culture thereof to the foregoing soybean extract may be about 1:1.5.

In another embodiment, in the probiotic composition of the present disclosure, the foregoing prebiotic may comprise a soybean extract and an apple extract. In this embodiment, the weight ratio of the foregoing probiotic of the present disclosure or the culture thereof, the foregoing soybean extract and the foregoing apple extract may be about 1-6:1-6:1-6, such as about 1-5:1-5:1-5, about 1.5-4:1.5-4:1.5-4, about 2-5:2-5:2-5, about 2.5-6:2.5-6:2.5-6, about 1:1:1, about 1:1.5:1.5, about 1:1.5:1, about 1:1:1.5, about 1.5:1:1, about 1:2:2, about 1:2:1, about 1:1:2, about 2:2:1, about 2:1:1, about 1:2.5:2.5, about 1:2.5:1, about 1:1:2.5, about 2.5:1:1, about 1:3:3, about 1:3:1, about 1:1:3, about 3:1:1, about 1:4:4, about 1:4:1, about 1:1:4, about 4:1:1, about 2:3:3, about 3:2:2, about 5:6:6, about 6:5:5, but it is not limited thereto. Moreover, in one specific embodiment of this embodiment, the weight ratio of the foregoing probiotic of the present disclosure or the culture thereof, the foregoing soybean extract and the foregoing apple extract may be about 1:1:1. In another specific embodiment of this embodiment, the weight ratio of the foregoing probiotic of the present disclosure or the culture thereto, the foregoing soybean extract and the foregoing apple extract may be about 1:1.5:1.5.

In addition, the probiotic composition of the present disclosure may be a food composition, a pharmaceutical composition, etc., but it is not limited thereto.

In one embodiment, the probiotic composition of the present disclosure may be a food composition. In this embodiment, the probiotic composition of the present disclosure may be prepared as an oral dosage form, but it is also not limited thereto.

In another embodiment, the probiotic composition of the present disclosure may be a pharmaceutical composition. In this embodiment, the probiotic composition of the present disclosure may also be prepared as an oral dosage form, but it is also not limited thereto.

The oral dosage form prepared by the probiotic composition of the present disclosure may include, but is not limited to, a powder, a granule, a tablet, a troche, a pill, a capsule, a lozenge, a solution, a suspension, an emulsion, a syrup, an elixir or a slurry, etc.

In one embodiment, the oral dosage form prepared by the probiotic composition of the present disclosure may be a powder. In another embodiment, the oral dosage form prepared by the probiotic composition of the present disclosure may be a capsule. In yet another embodiment, the oral dosage form prepared by the probiotic composition of the present disclosure may be a solution.

Moreover, in the embodiment in which the probiotic composition of the present disclosure may be a food composition, the probiotic composition of the present disclosure, in addition to the probiotic culture mentioned above and the prebiotic mentioned above, may further comprise an additive which is acceptable for food.

The foregoing additive which is acceptable for food may include, but is not limited to, a preservative, a bactericide, an antioxidant, a bleaching agent, a color retention agent, an expander, a coloring agent, a flavoring agent, a sweetener, a thickener (paste), a binder, an emulsifier, a carrier, etc., or any combination thereof.

Moreover, in the embodiment in which the probiotic composition of the present disclosure may be a pharmaceutical, the probiotic composition of the present disclosure, in addition to the probiotic culture mentioned above and the prebiotic mentioned above, may further comprise a pharmaceutically acceptable carrier or salt, but it is not limited thereto.

The pharmaceutically acceptable carrier mentioned above may comprise, but is not limited to, a solvent, a dispersion medium, a coating, an antibacterial and antifungal agent, or an isotonic and absorption delaying agent, etc.

which is suitable for pharmaceutical administration. The pharmaceutical composition can be formulated into dosage forms for different administration routes utilizing conventional methods.

Moreover, the pharmaceutically acceptable salt mentioned above may comprise, but is not limited to, salts including inorganic cation, such as alkali metal salts such as sodium salt, potassium salt or amine salt, such as alkaline-earth metal salt such as magnesium salt or calcium salt, such as the salt containing bivalent or quadrivalent cation such as zinc salt, aluminum salt or zirconium salt. In addition, the pharmaceutically acceptable salt may also be organic salt, such as dicyclohexylamine salt, methyl-D-glucamine, and amino acid salt such as arginine, lysine, histidine, glutamine, but it is not limited thereto.

## EXAMPLES

### A. Materials and Methods

### 1. Bacterial species identification

A strain isolated from the human intestine was subjected to genome-wide average nucleotide identity analysis based on the GenomesDB database using the software/tool for average nucleotide identity (ANI) calculation, JSpeciesWS online web server. The results are shown in Table 1 below.

Table 1

| Type strains | Size | GC [%] | ANIb value (%) |
|---|---|---|---|
| *Bifidobacterium breve* LMG 13208 | 2,263,780 | 58.88 | 97.93 |
| *Bifidobacterium breve* BBRI4 | 2,426,720 | 58.72 | 97.74 |
| *Bifidobacterium breve* BR3 | 2,426,006 | 59.09 | 97.81 |
| *Bifidobacterium breve* GED8481 | 2,356,336 | 58.66 | 97.75 |
| *Bifidobacterium breve* NCTC11815 [T] | 2,275,664 | 58.89 | 97.95 |
| *Bifidobacterium breve* 12L | 2,244,624 | 58.87 | 98.03 |
| *Bifidobacterium breve* 2L | 2,240,722 | 58.85 | 98.03 |
| *Bifidobacterium breve* 31L | 2,265,654 | 58.57 | 98.06 |
| *Bifidobacterium breve* 689b | 2,331,707 | 58.73 | 97.98 |
| *Bifidobacterium breve* ACS-071-V-Sch8b | 2,327,492 | 58.73 | 98.43 |
| *Bifidobacterium breve* CECT 7263 | 2,314,396 | 58.88 | 98.33 |
| *Bifidobacterium breve* DSM 20213 = JCM 1192 DSM 20213 | 2,257,131 | 58.87 | 97.94 |
| **Isolated strain of the present disclosure** | **2,460,420** | **59.1** | **100** |

According to the results of the genome-wide average nucleotide identity analysis, it is known that the whole genome sequence of this isolated strain is 97.74% to 98.43% similar to that of *Bifidobacterium breve* subspecies (as shown in Table 1). Therefore, this isolated strain is confirmed to be a novel *Bifidobacterium breve*.

The novel *Bifidobacterium breve* was deposited in the Bioresource Collection and Research Center (BCRC) of Food Industry Research and Development Institute (FIRDI), Taiwan, China on August 8, 2022, and the deposit number BCRC 911145 was obtained, and the novel *Bifidobacterium breve* was also deposited in the German Collection of Microorganisms and Cell Cultures GmbH (DSMZ) on September 7, 2022 and the deposit number DSM 34383 was obtained. This novel *Bifidobacterium breve* hereinafter will be referred to *Bifidobacterium breve* BCRC 911145.

### 2. Culturing of *Bifidobacterium breve* BCRC 911145 and acquisition of culture

### 2-1. Culturing of *Bifidobacterium breve* BCRC 911145

### 2-1-1. Preparation of 5 L fermenter

3 L of MRS broth (DeMan-Rogosa-Sharpe broth) was prepared (55 g/L) in a 5 L fermenter using commercially available food-grade MRS broth powder (manufacturer: STBIO MEDIA, INC.). It was necessary to confirm that the powder in the prepared medium was completely dissolved.

The foregoing 5 L fermenter containing the medium was sterilized (121°C, 30 minutes). After the sterilization was completed, the fermenter was aerated (3 L/minute) and cooled to 37°C.

**2-1-2. Fermentation in 5 L fermenter**

After the preparation of fermenter mentioned above was completed, the fermenter was aerated (3 L/minute) and the medium was stirred (100 rpm) at 37°C for 20 minutes. At the same time, the dissolved oxygen electrode was calibrated (100%).

*Bifidobacterium breve* BCRC 911145 could be implanted into the medium of the fermenter for performing fermentation only when the dissolved oxygen was less than 5%, and before implanting the bacterial strain, it was necessary to confirm that the fermenter was not contaminated.

The conditions for bacterial strain implantation and fermentation are as follows:

(1) Absorbance at 600nm ($OD_{600}$) of bacterial strain suspension for implantation in fermenter: 3.0-5.0;
(2) Amount of implanted bacteria: 5% (v/v);
(3) Dissolved oxygen: <1% (aerating with a small amount of nitrogen to maintain);
(4) Stirring speed: 25 rpm;
(5) Temperature: $37\pm1°C$;
(6) pH: $6.0\pm0.1$;
(7) Culturing time: about 6-8 hours (culturing until the absorbance at 600 nm ($OD_{600}$) of the cultured medium in the fermenter to be 3.0-4.0).

After fermentation was completed, samples were taken for inspection and photographed under a microscope to confirm the bacterial morphology and confirm bacterial strain activity.

After that, the fermenter was cooled (the temperature was set to 4°C, the actual temperature was less than 15°C).

**2.2 Acquisition of culture**

**2-2-1. Acquisition of viable bacteria of cultured *Bifidobacterium breve* BCRC 911145**

After the foregoing "2-1-2. Fermentation in 5 L fermenter" was completed, centrifugation was performed to remove the medium to obtain viable bacteria.

The centrifugation conditions are as follows:
10000 rpm, 10 minutes.

After centrifugation, the viable bacteria were lyophilized.

Afterwards, the lyophilized viable bacteria were pulverized to form viable bacteria powder.

**2-2-2. Acquisition of cultured supernatant of cultured *Bifidobacterium breve* BCRC 911145**

After the foregoing "2-1-2. Fermentation in 5 L fermenter" was completed, the fermenter was sterilized (steam sterilization for 30 minutes).

After cooling, centrifugation was performed to remove the bacterial cells and obtain the cultured supernatant.

The centrifugation conditions are as follows:
10000 rpm, 10 minutes.

**2-2-3. Acquisition of dead bacteria suspension of cultured *Bifidobacterium breve* BCRC 911145**

After the foregoing "2-1-2. Fermentation in 5 L fermenter" was completed, the fermenter was sterilized (steam sterilization for 30 minutes).

After cooling, centrifugation was performed to remove the medium and obtain the dead bacteria.

The centrifugation conditions are as follows:
5000 rpm, 10 minutes.

After centrifugation, water in an amount equal to the volume of the culture before centrifugation was added to the dead bacteria to form a dead bacteria suspension.

**3. Detection of glucagon-like peptide-1 (GLP-1) secreted by cells *in vitro***

3-1. Materials

(1) Cell line: NCI-H716 cells (ATCC, CCL-251);
(2) Medium:

RPMI 1640 Media (Gibco);
10% (v/v) Fetal bovine serum (Gibco);
2 mM L-Glutamine (Gibco);
1% (v/v) Penicillin-streptomycin (Gibco);

(3) Trypan blue dead cell stain (Lonza; Cat. 17-942E);
(4) PBS (Gibco);
(5) ELISA reader (BioTek);
(6) GLP-1 ELISA KIT (CEA804Mi).

### 3-2. Methods

(1) The cells were seeded in a 6-well cell culture plate (culture volume was 2 mL) at a density of $1\times10^5$ cells/well and cultured in a 37°C, 5% $CO_2$ incubator for 24 hours.
(2) Two days before the detection, $1\times10^5$ cells/well were seeded into a 24-well plate coated with Matrix gel, and the medium was replaced with DMEM complete medium and the cells were cultured for 24 hours;
(3) One day before the detection, the cell culture medium was changed to DMEM w/o glucose medium, and forskolin (final concentration in the medium was 1 $\mu$M) and 3-isobutyl-1-methylxanthine (IBMX) (final concentration in the medium was 1 $\mu$M) were added at the same time and co-cultured for 4 hours;
(4) The supernatant in each well of the culture plate was removed, then PBS containing 1 mM $CaCl_2$ (300 $\mu$L/well) was added to each well, and different samples to be tested and 1 $\mu$M forskolin and 1 $\mu$M 3-isobutyl-1-methylxanthine were added. After that, culturing was performed for 24 hours;
(5) After 24 hours of culturing, the supernatant was aspirated from each well, and the GLP-1 content in the supernatant was detected using GLP-1 ELISA KIT (CEA804Mi) to confirm the amount of GLP-1 secreted by the cells into the medium;
(6) Statistical analysis was performed using Excel with one-tailed Student's t-test (T-TEST) (compared to Mock, *: $p < 0.05$, **: $p < 0.01$, ***: $p < 0.001$).

### 4. Detection of PYY gene expression in cells *in vitro*

### 4-1. Materials and Instruments

(1) Cell line: NCI-H716 cells (ATCC, CCL-251);
(2) Medium:

89% (v/v) RPMI 1640 Media (Gibco);
10% (v/v) Fetal bovine serum (Gibco);
2 mM L-Glutamine (Gibco);
1% (v/v) Penicillin-streptomycin (Gibco);

(3) RNA Extraction Kit (Geneaid);
(4) SuperScript® III reverse transcriptase (Invitrogen);
(5) Primer set: Primer set for PYY gene and primer set for GAPDH gene as internal control;
(6) KAPA CYBR FAST qPCR Kits (2x) (KAPA Biosystems);
(7) StepOnePlus Real-Time PCR System (API).

### 4-2. Methods

### 4-2-1. Cell culture and pre-treatment

(1) The cells were seeded in a 6-well cell culture plate (culture volume was 2 mL) at a density of $1 \times 10^5$ cells/well and cultured in a 37°C, 5% $CO_2$ incubator for 24 hours.
(2) Afterwards, the cells were treated with different samples to be tested for 24 hours and 48 hours;
(3) The cells were harvested for performing gene expression analysis.

### 4-2-2. Gene expression analysis

(1) RNA was extracted from cells using RNA Extraction Kit (Geneaid);
(2) The above-mentioned RNA (2000 ng) was reverse transcribed into cDNA using SuperScript® III Reverse Transcriptase (Invitrogen);
(3) The cDNA was subjected to real-time polymerase chain reaction using KAPA SYBR FAST qPCR Kits (2x) (KAPA Biosystems) by StepOnePlus Real-Time PCR System to measure the expression level of the target gene;
(4) The melting curve during real-time polymerase chain reaction was analyzed;
(5) Relative quantification of gene expression was determined using the $2^{-\Delta\Delta C_T}$ method. The threshold cycle ($C_T$ value) of the GAPDH reference gene was used as the internal control and the simulation group, and the relative fold change was calculated according to the formulas shown in Formula (I) to Formula (III) below:

$$\Delta C_T = C_{T\ Target\ gene/Reference\ gene} - C_{T\ Internal\ control}$$

Formula (I);

$$\Delta\Delta C_T = \Delta C_{T\ Target\ gene} - \Delta C_{T\ Reference\ gene}$$

Formula (II);

$$Fold\ change = 2^{-\Delta\Delta C_{T\ Mean}}$$

Formula (III)

(6) The standard deviation of the relative expression levels of gene was calculated by STDEV in Excel;
(7) Statistical analysis was performed using Excel with one-tailed Student's t-test (T-TEST) (*: $p < 0.05$, **: $p < 0.01$, ***: $p < 0.001$).

### 5. Detection of the expressions of fat-related genes (CEBPA, UCP, ATGL, PLIN1, PPARG2 and LIPE(HSL)) in cells *in vitro*

### 5.1 Materials and Instruments

(1) Cell line: Mouse stroma bone marrow, stroma OP9 (BCRC; Cat. 60566);
(2) Medium:

Minimum essential medium α (MEMα) (Gibco; Cat.12000-022, or an item with the same function);
2% (v/v) Fetal bovine serum (Gibco; Cat. 10437-028);
1% (v/v) Antibiotics (Gibco; Cat.15240-062, or an item with the same function);

(3) 10X DPBS (Gibco; Cat. 14200-075, or an item with the same function);

(4) Trypan blue dead cell stain (Lonza; Cat. 17-942E, or an item with the same function);

(5) RB Buffer (Geneaid; Cat. RP050, or an item with the same function);

(6) 10X Trypsin solution (SIGMA; Cat.59427C, or an item with the same function);

(7) RNA Extraction Kit (Geneaid);

(8) SuperScript® III reverse transcriptase (Invitrogen);

(9) Primer set: Primer sets for each gene to be tested (CEBPA, UCP, ATGL, PLIN1, PPARG2 and LIPE(HSL)) and primer set for the GAPDH gene as an internal control;

(10) KAPA CYBR FAST qPCR Kits (2x) (KAPA Biosystems);

(11) StepOnePlus Real-Time PCR System (API).

### 5-2. Methods

### 5-2-1. Preparation of working reagents

(1) 1X DPBS: 10X DPBS was diluted 10-fold with sterilized ddH$_2$O;

(2) 1X Trypsin solution: 10X Trypsin solution was diluted 10-fold with 1X DPBS.

### 5-2-2. Cell culture and pre-treatment

(1) The cells were seeded in a 6-well cell culture plate at a density of $1\times10^5$ cells/well (culture volume was 2 mL) and cultured in a 37°C, 5% CO$_2$ incubator for 24 hours;

(2) After that, the cells were divided into Mock group and experimental groups (high-dose group and low-dose group), and after the addition of different samples to be tested, the cells were placed in a 37°C, 5% CO$_2$ incubator for treatment for 6 hours, 24 hours and 48 hours;

(3) After the treatment was completed, the medium was removed;

(4) the cells were washed with 1X DPBS (1 mL/well) and then the 1X DPBS was removed;

(5) Step (4) was repeated;

(6) RB Buffer (600 μL/well) was added to lyse the cells to obtain a cell lysate solution;

(7) The cell lysate solution was collected in a 1.5 mL microcentrifuge tube and stored in a -80°C refrigerator.

### 5-2-3. Gene expression analysis

(1) RNA was extracted from cells using RNA Extraction Kit (Geneaid);

(2) The above-mentioned RNA (2000 ng) was reverse transcribed into cDNA using SuperScript® III Reverse Transcriptase (Invitrogen);

(3) The cDNA was subjected to real-time polymerase chain reaction using KAPA SYBR FAST qPCR Kits (2x) (KAPA Biosystems) by StepOnePlus Real-Time PCR System to measure the expression level of the target gene;

(4) The melting curve during real-time polymerase chain reaction was analyzed;

(5) Relative quantification of gene expression was determined using the $2^{-\Delta\Delta C_T}$ method. The threshold cycle (C$_T$ value) of the GAPDH reference gene was used as the internal control and the simulation group, and the relative fold change was calculated according to the formulas shown in Formula (I) to Formula (III) above.

### 6. Detection of fat accumulation in cells *in vitro*

OP9 mouse embryonic stem cells were used as a cell platform to confirm whether the test samples in the experimental group have the effect of reducing oil droplet formation.

### 6-1. Materials

(1) Cell line: OP9 mouse embryonic stem cell (ATCC; Cat. CRL-2749);
(2) Medium:

79% (v/v) Minimum essential medium $\alpha$ (MEM$\alpha$) (Gibco; Cat. 12561072);
20% (v/v) Fetal bovine serum (Gibco; Cat. 10437-028);
1% (v/v) Penicillin-streptomycin (Gibco; Cat. 15140122);

(3) 10X DPBS (Gibco; Cat. 14200-075, or an item with the same function);
(4) Trypan blue dead cell stain (Lonza; Cat. 17-942E, or an item with the same function);
(5) 10X Trypsin solution (Gibco; Cat. 15400-054);
(6) Oil red O (Sigma; Cat. O0625-25G);
(7) Isopropanol (ECHO CHEMICAL CO., LTD.; Cat. 184130010);
(8) Formaldehyde (ECHO CHEMICAL CO., LTD.; Cat. 119690010).

## 6-2 Methods

### 6-2-1. Preparation of working reagents

(1) 1X DPBS: 10X DPBS was diluted 10-fold with sterilized ddH$_2$O;
(2) 1X Trypsin solution: 10X Trypsin solution was diluted 10-fold with 1X DPBS;
(3) 10% Formaldehyde: Formaldehyde was diluted with sterilized ddH$_2$O;
(4) 50% and 60% Isopropanol: Isopropanol was diluted with sterilized ddH$_2$O;
(5) Oil red O stock solution: Oil Red O was prepared with 100% isopropanol to an Oil Red O stock solution with a final concentration of 3 mg/mL;
(6) Oil red O working solution: Oil Red O stock solution was mixed with ddH$_2$O in a ratio of 3:2 to form a mixed solution. The mixed solution was then centrifuged at 1000 x g for 10 minutes and the supernatant was taken to a new 15 mL centrifuge tube for use.

### 6-2-2. Cell culture and pre-treatment

(1) The cells were seeded in a 24-well cell culture plate at a density of 8 x 10$^4$ cells/well (culture volume was 500 $\mu$L/well) and cultured in a CO$_2$ incubator for 7 days, with replacing the medium every 3 days;
(2) After 7 days, the formation of oil droplets in the cells was observed under a microscope to confirm cell differentiation;
(3) The medium in the culture plate was replaced with the medium for the experimental groups (containing the test sample) and the medium for the control group (Mock), and culturing was performed in a CO$_2$ incubator for 7-10 days, with replacing the medium every 3 days.

### 6-2-3. Determination of oil droplet content

(1) After the culture was completed, the liquid in the well plate was removed and the cells were rinsed twice with 1X DPBS;
(2) 10% methanol (1 mL/well) was added to the cells and the cells was fixed for 30 minutes at room temperature;
(3) The liquid in the well plate was removed and the cells were rinsed twice with 1X DPBS;
(4) The liquid in the well plate was removed and 60% isopropanol (1 mL/well) was added and let stand at room temperature for 1 minute;
(5) The liquid in the well plate was removed and Oil Red O working solution (1 mL/well) was added for staining for 1 hour at room temperature in the dark;
(6) The liquid in the well plate was removed and 60% isopropanol (1 mL/well) was added for destaining for 5 seconds;
(7) The liquid in the well plate was removed and ddH$_2$O (1 mL/well) was added;
(8) Observation and photographing were performed with a microscope;
(9) The liquid in the well plate was removed and 100% isopropanol was added, and the well plate was placed on an orbital shaker and shaken for 10 minutes to elute the dye;

(10) The liquid in the well plate was added to a 96-well plate (100 $\mu$L/well) and the absorbance value at a wavelength of 510 nm (O.D. 510) thereof was determined by a spectrophotometer;

(11) Statistical analysis was performed using Excel with one-tailed Student's t-test (T-TEST) (*: $p < 0.05$, **: $p < 0.01$, ***: $p < 0.001$).

### 7. *In vitro* cellular lipolysis assay

OP9 mouse embryonic stem cells were used as a cell platform to confirm the effect of the stimulation of the test sample in the experimental group on the glycerol content released into the medium during the OP9 adipogenic differentiation, so as to know whether the test sample has the effect of lipolysis.

### 7-1. Materials

(1) Cell line: OP9 mouse embryonic stem cell (ATCC; Cat. CRL-2749);
(2) Medium:

79% (v/v) Minimum essential medium $\alpha$ (MEM$\alpha$) (Gibco; Cat. 12561072);
20% (v/v) Fetal bovine serum (Gibco; Cat. 10437-028);
1% (v/v) Penicillin-streptomycin (Gibco; Cat. 15140122);

(3) 10X DPBS (Gibco; Cat. 14200-075, or an item with the same function);
(4) Trypan blue dead cell stain (Lonza; Cat. 17-942E, or an item with the same function);
(5) 10X Trypsin solution (Gibco; Cat. 15400-054);
(6) Glycerol cell-based assay kit (Cayman; Cat. 10011725):

Glycerol standard solution (Cayman; Cat. 10009952);
Free glycerol assay reagent (Cayman; Cat. 10009953);
Chloroquine positive control (25 mM) (Cayman; Cat. 10012670).

### 7-2. Methods

### 7-2-1. Preparation of working reagents

(1) 1X DPBS: 1X DPBS: 10X DPBS was diluted 10-fold with sterilized ddH$_2$O;
(2) 1X Trypsin solution: 10X Trypsin solution was diluted 10-fold with 1X DPBS.
(3) Free glycerol assay reagent: The vial with this label (Cat. 10009953) in the kit was opened and 5 mL of ddH$_2$O was added to dissolve the content in the vail to prepare the free glycerol assay reagent. The amount of one vial could supply 48 samples (half a 96-well plate). What prepared can be stored at 4°C for up to 2 weeks;
(4) Glycerol standard solution: The concentration of the glycerol stock solution in the vial with this label (Cat. 10009952) in the kit was 125 $\mu$g/mL. This stock solution was serially diluted. Eight clean 1.5 mL microcentrifuge tubes were individually written with numbers 1 through 8. 200 $\mu$L of glycerol stock solution was added to tube 1, and 100 $\mu$L of 1X DPBS was added to each of the remaining 7 tubes. 100 $\mu$L was then taken from tube 1 to tube 2, mixed well, and then 100 $\mu$L was taken from the second tube to tube 3, and this operation was repeated to perform serially dilution to tube 7 to respectively obtain glycerol solutions with the concentrations of 125 $\mu$g/mL (tube 1), 62.5 $\mu$g/mL (tube 2), 31.25 $\mu$g/mL (tube 3), 15.625 $\mu$g/mL (tube 4), 7.8125 $\mu$g/mL (tube 5), 3.90625 $\mu$g/mL (tube 6), 1.953125 $\mu$g/mL (tube 7), and 0 $\mu$g/mL (tube 8).

### 7-2-2. Cell culture and pre-treatment

(1) The cells were seeded in a 24-well cell culture plate at a density of 8 x 10$^4$ cells/well (culture volume was 500 $\mu$L/well) and cultured in a CO$_2$ incubator for 7 days, with replacing the medium every 3 days;

(2) After 7 days, the formation of oil droplets in the cells was observed under a microscope to confirm cell differentiation;

(3) The medium was replaced with the medium for the experimental groups (the test samples) and the medium for the control group (Mock), and culturing was performed in a $CO_2$ incubator for 7-10 days, with replacing the medium every 3 days.

### 7-2-3. Determination of glycerol content released into the medium

(1) After the culture was completed, the cell cultured medium of the last culture was collected;

(2) 25 $\mu$L of the cell cultured medium, glycerol standard solution with various concentrations and chloroquine positive control were separately added to a 96-well plate;

(3) The free glycerol assay reagent (100 $\mu$L/well) was added and reacted at room temperature for 15 minutes;

(4) The absorbance value at a wavelength of 540 nm (O.D. 540) of each well was determined by a spectrophotometer;

(5) Statistical analysis was performed using Excel with one-tailed Student's t-test (T-TEST) (*: $p < 0.05$, **: $p < 0.01$, ***: $p < 0.001$).

### 8. Detection of the expressions of blood lipids-related genes (CETP, SCARB1, ABCA1, LDLR) in cells *in vitro*

### 8.1 Materials and Instruments

(1) Cell line: Human hepatocellular carcinoma cell HepG2 (ATCC; Cat. HB-8065);

(2) Medium:

Dulbecco's modified Eagle's medium (DMEM) (Gibco; Cat. 11965-092);
10% (v/v) Fetal bovine serum (Gibco; Cat.10437-028, or an item with the same function);
1% (v/v) Penicillin-streptomycin (Gibco; Cat. 15140122);

(3) 10X DPBS (Gibco; Cat. 14200-075, or an item with the same function);

(4) Trypan blue dead cell stain (Lonza; Cat.17-942E, or an item with the same function);

(5) RB Buffer (Geneaid; Cat. RP050, or an item with the same function);

(6) 10X Trypsin solution (SIGMA; Cat.59427C, or an item with the same function);

(7) RNA Extraction Kit (Geneaid);

(8) SuperScript® III reverse transcriptase (Invitrogen);

(9) Primer set: Primer sets for each gene to be tested (CETP, SCARB1, ABCA1 and LDLR) and primer set for the GAPDH gene as an internal control;

(10) KAPA CYBR FAST qPCR Kits (2x) (KAPA Biosystems);

(11) StepOnePlus Real-Time PCR System (ABI).

### 8-2. Methods

### 8-2-1. Cell culture and pre-treatment

(1) The cells were seeded in a 6-well plate at a density of $1 \times 10^6$ cells/well and cultured overnight (n=3);

(2) Cells were treated with different samples to be tested for 6, 24, 48 hours;

(3) After removing the supernatant, the cells were washed once with 1X PBS;

(4) After the cells were lysed by adding 600 $\mu$L of RB Buffer, the cell lysate solution was collected in a centrifuge tube and stored in a -80°C refrigerator.

### 8-2-2. Gene expression analysis

(1) RNA was extracted from cells using RNA Extraction Kit (Geneaid);

(2) The above-mentioned RNA (2000 ng) was reverse transcribed into cDNA using SuperScript® III Reverse Transcriptase (Invitrogen);

(3) The cDNA was subjected to real-time polymerase chain reaction using KAPA SYBR FAST qPCR Kits (2x) (KAPA Biosystems) by StepOnePlus Real-Time PCR System to measure the expression level of the target gene;

(4) The melting curve during real-time polymerase chain reaction was analyzed;

(5) Relative quantification of gene expression was determined using the $2^{-\Delta\Delta C_T}$ method. The threshold cycle ($C_T$ value) of the GAPDH reference gene was used as the internal control and the simulation group, and the relative fold change was calculated according to the formulas shown in Formula (I) to Formula (III) above.

### 9. Detection of Glut4 expression level in cells *in vitro*

#### 9.1 Materials and Instruments

(1) Cell line: Human hepatocellular carcinoma cell HepG2 (ATCC; Cat. HB-8065);

(2) Medium:

Dulbecco's modified Eagle's medium (DMEM) (Gibco; Cat. 12100046);
10% Fetal bovine serum (Gibco; Cat.10437-028, or an item with the same function);
1% Antibiotics (Gibco; Cat.15240-062, or an item with the same function);
Sodium bicarbonate (Sigma; Cat.S5761-500G, or an item with the same function);
Sodium hydroxide (NaOH) (Sigma; Cat.221465-500G, or an item with the same function);
Hydrogen chloride (HCl) (Sigma; Cat.7647-01-0, or an item with the same function);

(3) 10X DPBS (Gibco; Cat. 14200-075, or an item with the same function);

(4) Fetal bovine serum (Gibco; Cat.10437-028, or an item with the same function);

(6) 10X Trypsin solution (Gibco; Cat.15400054, or an item with the same function);

(7) Alexa Fluor 488 goat anti-mouse IgG antibody (Invitrogen; Cat.A-11001, or an item with the same function);

(8) GLUT4 antibody (Invitrogen; Cat.MA5-17176, or an item with the same function);

(9) Insulin (Sigma; Cat.I9278-5ML, or an item with the same function);

(10) Trypan blue dead cell stain (Lonza; Cat.17-942E, or an item with the same function);

(11) Flow cytometer (BD, Accuri C6 Plus, or an item with the same function).

#### 9-2-1. Preparation of working reagents

(1) 1X DPBS: 10X DPBS was diluted 10-fold with sterilized ddH$_2$O;

(2) 1X Trypsin solution: 10X Trypsin solution was diluted 10-fold with pure water;

(3) 2% Fetal bovine serum solution: 1 mL Fetal bovine serum was mixed well with 49 mL 1X DPBS in a 50 mL centrifuge tube.

#### 9-2-2. Cell culture and pre-treatment

(1) The cells were seeded in a 6-well cell culture plate at a density of $1 \times 10^5$ cells/well (culture volume was 2 mL) and cultured in a 37°C, 5% CO$_2$ incubator for 24 hours;

(2) After that, the cells were divided into Mock group and experimental groups (high-dose group and low-dose group);

(3) Optional: The original medium was removed, and serum-free medium containing 1 μM insulin was added to the cells, and cultured in a 37°C, 5% CO$_2$ incubator for 72 hours. After that, the medium was removed;

(4) Medium containing different concentrations of test samples was added to the cells and then the cells were cultured in a 37°C, 5% CO$_2$ incubator for 24 hours;

(5) Optional: 0.5 μM Insulin was added to the cells and the cells were cultured in a 37°C, 5% CO$_2$ incubator for 24 hours;

(6) After treatment was completed, the medium was removed;

(7) The cells were washed with 1X DPBS (1 mL/well) and then the 1X DPBS was removed;

(8) Step (7) was repeated;

(9) 1X Trypsin solution (200 $\mu$L/well) was added, the cells were placed in a 37°C, 5% $CO_2$ incubator for protecting it from light for 5 minutes, the culture plate was tapped and detachment of the cells was confirmed;

(10) Medium (800 $\mu$L/well) was added to terminate the action of trypsin;

(11) The cell suspension in each well was transferred to separate microcentrifuge tubes and centrifuged at 400 x g for 5 minutes to remove the supernatant;

(12) 200 $\mu$L 1X DPBS was added to each tube to wash the cells and then the tube was centrifuged at 400 x g for 5 minutes to remove the supernatant;

(13) 200 $\mu$L of 2% Fetal bovine serum solution was added to each tube and mixed well to fix the cells, and the cells were cultured at room temperature for 30-60 minutes in the dark;

(14) Each tube was centrifuged at 400 x g for 5 minutes to remove the supernatant;

(15) 200 $\mu$L 1X DPBS and 1 $\mu$L GLUT4 antibody (1:200) were added to each tube and mixed well, and the cells are cultured at room temperature for 30 minutes in the dark;

(16) Each tube was centrifuged at 400 x g for 5 minutes to remove the supernatant;

(17) 200 $\mu$L 1X DPBS was added to each tube to wash the cells and then the tube was centrifuged at 400 x g for 5 minutes to remove the supernatant;

(18) Step (17) was repeated;

(19) 200 $\mu$L 1X DPBS and 1 $\mu$L Alexa Fluor 488 goat anti-mouse IgG secondary antibody (1:200) were added to each tube and mixed well, and the cell were cultured at room temperature for 10 minutes in the dark;

(20) Each tube was centrifuged at 400 x g for 5 minutes to remove the supernatant;

(21) 200 $\mu$L 1X DPBS was added to each tube to wash the cells and then the tube was centrifuged at 400 x g for 5 minutes to remove the supernatant;

(22) Step (21) was repeated;

(23) 200 $\mu$L 1X DPBS was added to each tube to resuspend the cells;

(24) The fluorescence intensity of fluorescein isothiocyanate (FITC) was analyzed by flow cytometry;

(25) Statistical analysis was performed using Excel with one-tailed Student's t-test (T-TEST) (*: $p < 0.05$, **: $p < 0.01$, ***: $p < 0.001$).

## 10. Preparation of soybean extract

FUJI OIL CO., LTD, Japan was commissioned to produce the soybean extract.

The main processes are shown in sequence as follows:

(1) Soy protein isolation;
(2) Enzymatic hydrolysis;
(3) Isolation;
(4) Sterilization;
(5) Filtration;
(6) Spray drying; and
(7) Obtaining products.

## 11. Preparation of apple extract

The main processes of the apple extract are shown in sequence as follows:

(1) Apples were coarsely chopped (coarsely chopped hole size: 12 mm);

(2) The coarsely chopped apples were extracted with RO water to obtain a crude extract. The extraction conditions are as follows:

Weight ratio of coarsely chopped apples to RO water:
1:6;
Extraction temperature: 85$\pm$5°C;
Extraction time: 60 minutes;
Reflux pump 3.0$\pm$0.5kg/cm$^2$;

(3) The crude extract was filtered (pore size: 400 mesh) and sampled for testing after filtration (Brix: 8.0$\pm$0.5 (20°C));

(4) The filtrate was concentrated and sampled to confirm whether the concentrated liquid meets the predetermined specifications (Brix sugar: 8.0±0.5; CA: 0.3±0.2 (20°C); pH: 2.7±1.0 (20°C));
(5) After reaching the specifications, the concentrated liquid could be used as the apple extract used in the present disclosure.

**B. Ingredient analysis**

**1. Analysis of short-chain fatty acids (SCFA) contained in the cultured supernatant of cultured *Bifidobacterium breve* BCRC 911145**

It is currently known that short-chain fatty acids stimulate the secretion of glucagon-like peptide-1 (GLP-1). Therefore, the short-chain fatty acids contained in the cultured supernatant of cultured *Bifidobacterium breve* BCRC 911145 were analyzed to learn the potential of the cultured supernatant of cultured *Bifidobacterium breve* BCRC 911145 to stimulate the secretion of glucagon-like peptide-1 (GLP-1).
The results are shown in Table 2.

Table 2: Short-chain fatty acids (SCFAs) contained in the cultured supernatant of cultured *Bifidobacterium breve* BCRC 911145

| Short-chain fatty acids | C1 | C2 | C3 | C4 | isoC4 | isoC5 |
|---|---|---|---|---|---|---|
| | Formic acid | Acetic acid | Propionic acid | Butyric acid | Isobutyric acid | 3-Methylbutyric acid |
| (μM) | 11121.571 | 303688.219 | 790.959 | 178.32 | 88.731 | 10.291 |

According to Table 2, it is known that the cultured supernatant of cultured *Bifidobacterium breve* BCRC 911145 contains at least short-chain fatty acids such as formic acid, acetic acid, propionic acid, butyric acid, isobutyric acid and 3-methylbutyric acid, and therefore should have the ability to stimulate the secretion of glucagon-like peptide-1.

**C. *In vitro* experiments**

**1. Confirmation of the effects of cultured supernatant and dead bacteria suspension in promoting the secretion of glucagon-like peptide-1 (GLP-1) and reducing appetite**

Glucagon-like peptide-1 (GLP-1) is the most important incretin in the human body, which can promote insulin secretion and inhibit glucagon secretion, and have the effect of regulating blood glucose, reducing appetite, and controlling body weight, while PYY gene is an appetite suppression-related gene.
The effects of *Bifidobacterium breve* BCRC 911145 in appetite control, weight control and blood glucose modulation were evaluated by analyzing the effects of cultured supernatant and dead bacteria suspension of *Bifidobacterium breve* BCRC 911145 on glucagon-like peptide-1 (GLP-1) secretion and PYY gene expression.
According to the methods described in **"3. Detection of glucagon-like peptide-1 (GLP-1) secreted by cells *in vitro"*** and **"4. Detection of PYY gene expression in cells *in vitro"*** in "A. Materials and Methods" above, the effects of cultured supernatant and dead bacteria suspension of *Bifidobacterium breve* BCRC 911145 (tested with NCI-H716 cells) on glucagon-like peptide-1 secretion and PYY gene expression were confirmed, respectively.

FIG. 1A and Table 3 show the relative amount of glucagon-like peptide-1 (GLP-1) secreted into the medium by the cells treated with the cultured supernatant of *Bifidobacterium breve* BCRC 911145 (the final concentration of the cultured supernatant of *Bifidobacterium breve* BCRC 911145 in the medium for culturing the cells is 0.25%).

FIG. 1B and Table 4 show the relative expression of PYY gene in cells treated with the cultured supernatant of *Bifidobacterium breve* BCRC 911145 (cells are cultured for 48 hours, the final concentration of the cultured supernatant of *Bifidobacterium breve* BCRC 911145 in the medium for culturing cells is 0.5%).

FIG. 2A and Table 5 show the relative amount of glucagon-like peptide-1 (GLP-1) secreted into the medium by the cells treated with the dead bacteria suspension of *Bifidobacterium breve* BCRC 911145 (the final concentration of the dead bacteria suspension of *Bifidobacterium breve* BCRC 911145 in the medium for culturing the cells is 0.125%).

FIG. 2B and Table 6 show the relative expression of PYY gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing cells is 0.125%).

Table 3: Relative amount of glucagon-like peptide-1 (GLP-1) secreted into the medium by the cells treated with the cultured supernatant of *Bifidobacterium breve* BCRC 911145 (the final concentration of the cultured supernatant of *Bifidobacterium breve* BCRC 911145 in the medium for culturing the cells is 0.25%)

| | | Relative content of GLP-1 | Standard deviation | T-TEST |
|---|---|---|---|---|
| Mock | Mock | 100 | 5.667436084 | |
| Glucose (Positive control) | 30 mM Glucose | 138.5623 | 3.73147136 | *** |
| *Bifidobacterium breve* BCRC 911145 | Cultured supernatant 0.25% | 151.7905 | 2.109478447 | *** |

Compared to the Mock group, ***: $p < 0.001$.

Table 4: Relative expression of PYY gene in cells treated with the cultured supernatant of *Bifidobacterium breve* BCRC 911145 (cells are cultured for 48 hours, the final concentration of the cultured supernatant of *Bifidobacterium breve* BCRC 911145 in the medium for culturing cells is 0.5%)

| | Relative expression of PYY gene | Standard deviation |
|---|---|---|
| Mock | 1 | 0 |
| *Bifidobacterium breve* BCRC 911145 cultured supernatant 0.5% | 1.797643198 | 0.359497822 |

Table 5: Relative amount of glucagon-like peptide-1 (GLP-1) secreted into the medium by the cells treated with the dead bacteria suspension of *Bifidobacterium breve* BCRC 911145 (the final concentration of the dead bacteria suspension of *Bifidobacterium breve* BCRC 911145 in the medium for culturing the cells is 0.125%)

| | | Relative content of GLP-1 | Standard deviation | T-TEST |
|---|---|---|---|---|
| Mock | Mock | 100 | 2.897637825 | |
| Glucose (Positive control) | 30 mM Glucose | 133.2586786 | 13.31189218 | * |
| *Bifidobacterium breve* BCRC 911145 | Dead bacteria suspension 0.125% | 120.4927212 | 10.28495385 | * |
| Compared to the Mock group, **: $p < 0.05$. | | | | |

Table 6: Relative expression of PYY gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing cells is 0.125%

|  | Relative expression of PYY gene | Standard deviation |
|---|---|---|
| Mock | 1 | 0 |
| *Bifidobacterium breve* BCRC 911145 dead bacteria suspension 0.125% | 1.484153968 | 0.094467242 |

Based on the results mentioned above, it is know that both the cultured supernatant and the dead bacteria suspension of *Bifidobacterium breve* BCRC 911145 can effectively promote cells to secrete glucagon-like peptide-1 (GLP-1) and effectively enhance the expression of PYY gene in cells.

Specifically, the cultured supernatant of *Bifidobacterium breve* BCRC 911145 can promote the secretion of glucagon-like peptide-1 (GLP-1), with the secretion amount increased by 51.8%. At the same time, it can effectively increase the expression of the appetite suppression-related gene, PYY gene, with the expression increased by up to 79.8%.

Similarly, the dead bacteria suspension of *Bifidobacterium breve* BCRC 911145 can also promote the secretion of glucagon-like peptide-1 (GLP-1), with the secretion amount increased by 20.5%. At the same time, it can effectively increase the expression of the appetite suppression-related gene, PYY gene, with the expression increased by up to 48.4%.

In summary, both the cultured supernatant and the dead bacteria suspension of *Bifidobacterium breve* BCRC 911145 can be used for applications related to effects of appetite control, weight control and/or blood glucose modulation, etc.

**2. Confirmation of the effect of cultured supernatant in inhibiting adipocyte maturation and reducing fat accumulation**

According to the methods described in **"5. Detection of the expression of fat-related genes (CEBPA, UCP, ATGL, PLIN1, PPARG2 and LIPE(HSL)) in cells *in vitro***" and **"6. Detection of fat accumulation in cells *in vitro***" in "A. Materials and Methods" above, the effects of cultured supernatant of *Bifidobacterium breve* BCRC 911145 on C/EBPA gene expression and fat accumulation were confirmed.

C/EBPA gene is a key regulatory gene for energy homeostasis and lipid storage in the body. Its low expression will terminate the differentiation of lipid droplet cells.

The abilities of *Bifidobacterium breve* BCRC 911145 in inhibiting adipocyte maturation and reducing fat accumulation were evaluated by analyzing the effects of cultured supernatant of *Bifidobacterium breve* BCRC 911145 on C/EBPA gene expression and fat accumulation.

The results are shown in Table 7 and Table 8 and FIG. 3A and FIG. 3B.

Table 7 and FIG. 3A show the relative expression of C/EBPA gene in cells treated with the cultured supernatant of *Bifidobacterium breve* BCRC 911145 (cells are cultured for 6 hours, the final concentration of the cultured supernatant of *Bifidobacterium breve* BCRC 911145 in the medium for culturing cells is 0.25%).

Table 8 and FIG. 3B show the Oil red (lipid) content in cells treated with the cultured supernatant of *Bifidobacterium breve* BCRC 911145.

Table 7: Relative expression of C/EBPA gene in cells treated with the cultured supernatant of *Bifidobacterium breve* BCRC 911145 (cells are cultured for 6 hours, the final concentration of the cultured supernatant of *Bifidobacterium breve* BCRC 911145 in the medium for culturing cells is 0.25%

|  | **Relative expression level of C/EBPA gene** | **Standard deviation** |
|---|---|---|
| Mock | 1 | 0 |
| *Bifidobacterium breve* BCRC 911145 cultured supernatant 0.25% | 0.165920988 | 0.007281205 |

Table 8: Oil red (lipid) content in cells treated with the cultured supernatant of *Bifidobacterium breve* BCRC 911145 (the final concentration of the cultured supernatant of *Bifidobacterium breve* BCRC 911145 in the medium for culturing cells is 0.25%)

| | Oil red (lipid) content in cells | Standard deviation |
|---|---|---|
| Mock | 100 | 3.79 |
| *Bifidobacterium breve* BCRC 911145 cultured supernatant 0.25% | 78.97 | 1.24 |

According to the results mentioned above, it is known that the cultured supernatant of *Bifidobacterium breve* BCRC 911145 is capable of effectively inhibiting the expression of C/EBPA gene, i.e., inhibiting adipocyte maturation, with an inhibition rate as high as 83.4%, and successfully reducing the fat accumulation by 21.0%.
In summary, the cultured supernatant of *Bifidobacterium breve* BCRC 911145 can be used for applications related to fat reduction, etc.

**3. Confirmation of the effects of cultured supernatant and dead bacteria suspension in promoting fat burning and/or lipolysis**

According to the methods described in **"5. Detection of the expression of fat-related genes (CEBPA, UCP, ATGL, PLIN1, PPARG2 and LIPE(HSL)) in cells *in vitro*"** and **"7. *In vitro* cellular lipolysis assay"** in **"A. M**aterials and Methods" above, the effects of cultured supernatant of *Bifidobacterium breve* BCRC 911145 on UCP1 gene and UCP2 gene expressions and lipolysis were confirmed.
Elevated expressions of UCP1 gene and UCP2 gene contribute to the conversion of adipocytes and promote fat burning.
The potential of *Bifidobacterium breve* BCRC 911145 in promoting fat burning and lipolysis was evaluated by analyzing the effects of cultured supernatant and dead bacteria suspension of *Bifidobacterium breve* BCRC 911145 on UCP1 gene and UCP2 gene expressions and the effect of cultured supernatant of *Bifidobacterium breve* BCRC 911145 on lipolysis.
The results are shown in Table 9, Table 10 and Table 11, and FIG. 4A, FIG. 4B and FIG. 5.
Table 9 and FIG. 4A show the relative expressions of UCP1 gene and UCP2 gene in cells treated with the cultured supernatant of *Bifidobacterium breve* BCRC 911145 (cells are cultured for 24 hours, the final concentration of the cultured supernatant of *Bifidobacterium breve* BCRC 911145 in the medium for culturing cells is 0.125%).
Table 10 and FIG. 4B show the amount of glycerol in the cultured medium of cells treated with the cultured supernatant of *Bifidobacterium breve* BCRC 911145 (the final concentration of the cultured supernatant of *Bifidobacterium breve* BCRC 911145 in the medium for culturing cells is 0.25%).
Table 11 and FIG. 5 show the relative expressions of UCP1 gene and UCP2 gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing cells is 0.25%).

Table 9: Relative expressions of UCP1 gene and UCP2 gene in cells treated with the cultured supernatant of *Bifidobacterium breve* BCRC 911145 (cells are cultured for 24 hours, the final concentration of the cultured supernatant of *Bifidobacterium breve* BCRC 911145 in the medium for culturing cells is 0.125%)

| | Relative expression level | |
|---|---|---|
| Group/Gene | UCP1 | UCP2 |
| Mock | 1 | 1 |
| *Bifidobacterium breve* BCRC 911145 cultured supernatant 0.125% | 5.350352 | 2.839444 |
| | Standard deviation | |
| Mock | 0 | 0 |
| *Bifidobacterium breve* BCRC 911145 cultured supernatant 0.125% | 1.793952 | 0.277548 |

Table 10: Amount of glycerol in the cultured medium of cells treated with the cultured supernatant of *Bifidobacterium breve* BCRC 911145 (the final concentration of the cultured supernatant of *Bifidobacterium breve* BCRC 911145 in the medium for culturing cells is 0.25%)

| | Amount of glycerol in the cultured medium (%) | Standard deviation | T-TEST |
|---|---|---|---|
| Mock | 100.00 | 8.05 | |
| *Bifidobacterium breve* BCRC 911145 cultured supernatant 0.25% | 120.56 | 4.25 | * |
| Compared to the Mock group, **: $p < 0.05$. | | | |

Table 11: Relative expressions of UCP1 gene and UCP2 gene in cells treated with the dead bacteria suspension of *Bifidobacterium breve* BCRC 911145 (cells are cultured for 24 hours, the final concentration of the dead bacteria suspension of *Bifidobacterium breve* BCRC 911145 in the medium for culturing cells is 0.25%)

| | Relative expression level | |
|---|---|---|
| Group/Gene | UCP1 | UCP2 |
| Mock | 1 | 1 |
| *Bifidobacterium breve* BCRC 911145 dead bacteria suspension 0.25% | 2.332464 | 2.100877 |
| | Standard deviation | |
| Mock | 0 | 0 |
| *Bifidobacterium breve* BCRC 911145 dead bacteria suspension 0.25% | 0.099721 | 0.195996 |

Based on the results mentioned above, it is known that both the cultured supernatant and the dead bacteria suspension of *Bifidobacterium breve* BCRC 911145 can effectively promote fat burning and have the effect of reducing fat.

Specifically, the cultured supernatant of *Bifidobacterium breve* BCRC 911145 can effectively increase the expressions of fat burning related genes, UCP1 gene and UCP2 gene, with the highest increase rate reaching about 4.35 times, and at the same time can increase the lipolysis efficiency by about 20.6%.

Similarly, the dead bacteria suspension of *Bifidobacterium breve* BCRC 911145 can also effectively increase the expressions of fat burning related genes, UCP1 gene and UCP2 gene, with the highest increase rate reaching about 1.3 times.

In summary, both the cultured supernatant and the dead bacteria suspension of *Bifidobacterium breve* BCRC 911145 can be used for applications related to fat reduction, etc.

**4. Confirmation of the effects of cultured supernatant** and **dead bacteria suspension in metabolizing fat**

According to the methods described in **"5. Detection of the expression of fat-related genes (CEBPA, UCP, ATGL, PLIN1, PPARG2 and LIPE(HSL)) in cells *in vitro"*** in "A. Materials and Methods" above, the effects of cultured supernatant of *Bifidobacterium breve* BCRC 911145 on fat-related gene expressions were confirmed.

ATGL is involved in the first step of fat metabolism in lipid droplet cells, which can hydrolyze triglycerides. PLIN1 gene regulates fat metabolism in lipid droplet cells, and its low expression will promote fat metabolism in cells. PPARG2 regulates the β-oxidation process of fatty acids, and its low expression will promote fat metabolism. LIPE (HSL) hydrolyzes triglycerides into free fatty acids, which is responsible for converting cholesterol esters into free cholesterol. CEBPA terminates the differentiation of lipid droplet cells and is one of the key regulatory genes for energy homeostasis and lipid storage in the body.

The effect of *Bifidobacterium breve* BCRC 911145 on promoting fat metabolism was evaluated by analyzing the effects of the cultured supernatant and dead bacteria suspension of *Bifidobacterium breve* BCRC 911145 on the expression of each of the above genes.

The results are shown in Table 12 and Table 13, and FIGS. 6A to 6D and FIGS. 7A to 7D.

Table 12 and FIGS. 6A to 6D show the relative expressions of ATGL gene, PLIN1 gene, PPARG2 gene and LIPE (HSL) gene in cells treated with the cultured supernatant of *Bifidobacterium breve* BCRC 911145.

Table 13 and FIGS. 7A to 7D show the relative expressions of ATGL gene, PLIN1 gene, PPARG2 gene and CEBPA gene in cells treated with the dead bacteria suspension of *Bifidobacterium breve* BCRC 911145.

Table 12: Relative expressions of ATGL gene, PLIN1 gene, PPARG2 gene and LIPE (HSL) gene in cells treated with the cultured supernatant of *Bifidobacterium breve* BCRC 911145

| Culture time | Gene \ Group | Relative expression level | | | |
|---|---|---|---|---|---|
| | | ATGL | PLIN1 | PPARG2 | LIPE (HSL) |
| | Mock | 1 | 1 | 1 | 1 |
| 6 hours | *Bifidobacterium breve* BCRC 911145 cultured supernatant 0.25% | | 0.177391 | | |
| 24 hours | *Bifidobacterium breve* BCRC 911145 cultured supernatant 0.125% | | | 0.187532 | |
| 48 hours | *Bifidobacterium breve* BCRC 911145 cultured supernatant 0.125% | 1.387792 | | | 4.383512 |
| | | **Standard deviation** | | | |

| | | | | | |
|---|---|---|---|---|---|
| | Mock | 0 | 0 | 0 | 0 |
| 6 hours | *Bifidobacterium breve* BCRC 911145 cultured supernatant 0.25% | | 0.019206 | | |
| 24 hours | *Bifidobacterium breve* BCRC 911145 cultured supernatant 0.125% | | | 0.054401 | |
| 48 hours | *Bifidobacterium breve* BCRC 911145 cultured supernatant 0.125% | 0.230635 | | | 0.607965 |

Table 13: Relative expressions of ATGL gene, PLIN1 gene, PPARG2 gene and CEBPA gene in cells treated with the cultured supernatant of *Bifidobacterium breve* BCRC 911145

| Culture time | Group/Gene | Relative expression level | | | |
|---|---|---|---|---|---|
| | | ATGL | PLIN1 | PPARG2 | CEBPA |
| 6 hours | Mock | 1 | 1 | 1 | 1 |
| 24 hours | *Bifidobacterium breve* BCRC 911145 dead bacteria suspension 0.125% | | | | 0.642016 |
| 24 hours | *Bifidobacterium breve* BCRC 911145 dead bacteria suspension 0.25% | 1.067559 | 0.770049 | 0.889962 | |
| | | Standard deviation | | | |
| 6 hours | Mock | 0 | 0 | 0 | 0 |
| 24 hours | *Bifidobacterium breve* BCRC 911145 dead bacteria suspension 0.125% | | | | 0.044377 |
| 24 hours | *Bifidobacterium breve* BCRC 911145 dead bacteria suspension 0.25% | 0.042945 | 0.052777 | 0.024701 | |

Based on the results mentioned above, it is known that both the cultured supernatant and the dead bacteria suspension of *Bifidobacterium breve* BCRC 911145 can effectively promote fat metabolism and have the effect of reducing fat.

Specifically, the cultured supernatant of *Bifidobacterium breve* BCRC 911145 can effectively promote fat metabolism, with the highest increase rate reaching about 3.39 times.

Similarly, the dead bacteria suspension of *Bifidobacterium breve* BCRC 911145 can also effectively promote fat metabolism, with an average increase rate of about 10%.

In summary, both the cultured supernatant and the dead bacteria suspension of *Bifidobacterium breve* BCRC 911145 can be effectively used for applications related to fat reduction, etc.

**5. Confirmation of the effects of cultured supernatant** and **dead bacterial suspension in modulating blood lipids** and **promoting high-density lipoprotein cholesterol (HDL-C)**

According to the methods described in **"8. Detection of the expression of blood lipids-related genes (CETP, SCARB1, ABCA1, LDLR) in cells *in vitro"*** in "A. Materials and Methods" above, the effects of cultured supernatant and dead bacteria suspension of *Bifidobacterium breve* BCRC 911145 on blood lipids-related gene expressions were confirmed.

CETP gene, SCARB1 gene, and ABCA1 gene are related to the synthesis of high-density lipoprotein cholesterol (HDL-C), while the LDLR gene is related to the metabolism of low-density lipoprotein cholesterol (LDL-C).

The effects of *Bifidobacterium breve* BCRC 911145 in regulating blood lipids was evaluated by analyzing the effects of cultured supernatant and dead bacteria suspension of *Bifidobacterium breve* BCRC 911145 on the expression of each of the above genes.

The results are shown in Table 14 and Table 15, and FIGS. 8A to 8D and FIG. 9.

Table 14 and FIGS. 8A to 8D show the relative expressions of CETP gene, SCARB1 gene, ABCA1 gene and LDLR gene in cells treated with the cultured supernatant of *Bifidobacterium breve* BCRC 911145.

Table 15 and FIG. 9 show the relative expressions of CETP gene, SCARB1 gene and ABCA1 gene in cells treated with the dead bacteria suspension of *Bifidobacterium breve* BCRC 911145.

Table 14: Relative expressions of CETP gene, SCARB1 gene, ABCA1 gene and LDLR gene in cells treated with the cultured supernatant of *Bifidobacterium breve* BCRC 911145

| Culture time | Gene Group | Relative expression level | | | |
|---|---|---|---|---|---|
| | | CETP | SCARB1 | ABCA1 | LDLR |
| 6 hours | Mock | 1 | 1 | 1 | 1 |
| 6 hours | *Bifidobacterium breve* BCRC 911145 cultured supernatant 0.5% | 1.724851 | 1.179249 | 4.069225 | |
| 48 hours | *Bifidobacterium breve* BCRC 911145 cultured supernatant 0.5% | | | | 10.20103 |
| | | Standard deviation | | | |
| 6 hours | Mock | 0 | 0 | 0 | 0 |
| 6 hours | *Bifidobacterium breve* BCRC 911145 cultured supernatant 0.5% | 0.132365 | 0.12391 | 0.442441 | |
| 48 hours | *Bifidobacterium breve* BCRC 911145 cultured supernatant 0.5% | | | | 2.247979 |

Table 15: Relative expressions of CETP gene, SCARB1 gene and ABCA1 gene in cells treated with the dead bacteria suspension of *Bifidobacterium breve* BCRC 911145

| Culture time | Gene Group | Relative expression level | | |
|---|---|---|---|---|
| | | CETP | SCARB1 | APA1 |
| 6 hours | Mock | 1 | 1 | 1 |
| 6 hours | *Bifidobacterium breve* BCRC 911145 dead bacteria suspension 0.125% | 1.896516 | 1.3157348 | 1.621850302 |
| | | Standard deviation | | |
| 6 hours | Mock | 0 | 0 | 0 |
| 6 hours | *Bifidobacterium breve* BCRC 911145 dead bacteria suspension 0.125% | 0.153532 | 0.1588998 | 0.054549114 |

Based on the results mentioned above, it is known that both the cultured supernatant and the dead bacteria suspension of *Bifidobacterium breve* BCRC 911145 can effectively modulate blood lipids and promote the synthesis of high-density lipoprotein cholesterol.

Specifically, the cultured supernatant of *Bifidobacterium breve* BCRC 911145 can effectively promote the synthesis of high-density lipoprotein cholesterol, with the increase rate reaching up to about 9.2 times, and can also promote the metabolism of low-density lipoprotein cholesterol.

Similarly, the dead bacteria suspension of *Bifidobacterium breve* BCRC 911145 can also effectively promote fat metabolism and the synthesis of high-density lipoprotein cholesterol, with the increase rate reaching up to about 89.7%.

### 6. Confirmation of the effects of cultured supernatant and dead bacteria suspension in modulating blood glucose

According to the methods described in **"9. Detection of Glut4 expression in cells *in vitro"*** above, the effects of cultured supernatant and dead bacteria suspension of *Bifidobacterium breve* BCRC 911145 on blood glucose-related gene expression were confirmed.

It is currently known that glucose transporter type 4 (GLUT4) is a member of the family of integral membrane glucose transporters (GLUT). It is expressed in muscle, fat and liver tissues, and has the function of balancing blood glucose by the drawing effect on glucose to bring glucose into cells for use.

The effect of *Bifidobacterium breve* BCRC 911145 on modulating blood glucose was evaluated by analyzing the effects of cultured supernatant and dead bacteria suspension of *Bifidobacterium breve* BCRC 911145 on the expression of glucose transporter type 4 (GLUT4).

The results are shown in Table 16 and Table 17, and FIG. 10 and FIG. 11.

Table 16 and FIG. 10 show the relative expression of glucose transporter type 4 (GLUT4) in cells treated with the cultured supernatant of *Bifidobacterium breve* BCRC 911145.

Table 17 and FIG. 11 show the relative expression of glucose transporter type 4 (GLUT4) in cells treated with the dead bacteria suspension of *Bifidobacterium breve* BCRC 911145.

Table 16: Relative expression of glucose transporter type 4 (GLUT4) in cells treated with the cultured supernatant of *Bifidobacterium breve* BCRC 911145

| | Relative expression of glucose transporter type 4 (GLUT4) (%) | Standard deviation | T-TEST |
|---|---|---|---|
| Mock | 100.00 | 5.671702896 | |
| *Bifidobacterium breve* BCRC 911145 cultured supernatant 0.25% | 120.40 | 3.591077438 | ** |
| Compared to the Mock group, **: *p* < 0.01. | | | |

Table 17: Relative expression of glucose transporter type 4 (GLUT4) in cells treated with the dead bacteria suspension of *Bifidobacterium breve* BCRC 911145

| | Relative expression of glucose transporter type 4 (GLUT4) | Standard deviation | T-TEST |
|---|---|---|---|
| Mock | 1 | 0 | |
| *Bifidobacterium breve* BCRC 911145 dead bacteria suspension 0.25% | 2.156835291 | 0.629533696 | *** |
| Compared to the Mock group, ***: *p* < 0.001. | | | |

Based on the results mentioned above, both the cultured supernatant and the dead bacteria suspension of *Bifidobacterium breve* BCRC 911145 can effectively modulate blood glucose.

Specifically, the cultured supernatant of *Bifidobacterium breve* BCRC 911145 can increase the expression of glucose transporter type 4 (GLUT4) by about 20.4%.

Similarly, the dead bacteria suspension of *Bifidobacterium breve* BCRC 911145 can increase the expression of glucose transporter type 4 (GLUT4) by about 116%.

**D. Human trials**

**D-1. Efficacy test of viable bacteria powder**

Aims of the experiment: To test the weight loss effect of *Bifidobacterium breve* BCRC 911145

Experimental content:

1. Dosage form and dosage: *Bifidobacterium breve* BCRC 911145 viable bacteria capsule (100 mg viable bacteria powder/capsule, equivalent to $1 \times 10^9$ CFU/capsule), 1 capsule per day after dinner.
2. Experimental Design:
One viable bacteria capsule was taken daily for 4 consecutive weeks and the tests were performed before and 4 weeks after administration.
3. Conditions for subject:
Healthy adults over 20 years of age, known to have >25% body fat in males; >30% body fat in females.
4. Number of subjects: 9 persons.
5. Test Items:

(1) Survey-Appetite Status;

(2) Blood biochemical indexes (total cholesterol, HDL, LDL, LDL/HDL and fasting blood glucose) (Le Zen Reference Lab was entrusted for testing).

The results are shown in FIG. 12, FIG. 13, FIG. 14A and FIG. 14B, and Table 18.

FIG. 12 shows the results of the survey on the appetite status of the subjects.

Based on FIG. 12, it is known that after continuously taking *Bifidobacterium breve* BCRC 911145 viable bacteria capsules for 4 weeks, sporadic food cravings can be significantly reduced by 25.9% and hunger can be significantly reduced by 20.4%. In addition, the survey results also show that the proportion of respondents whose sporadic food cravings and hunger symptoms are improved is 62.5% and 87.5% of the total number of people, respectively.

Table 18: Blood biochemical indexes of subjects

| Glucose | Mean | Standard deviation | % | P value | Total number of respondents | 9 |
|---|---|---|---|---|---|---|
| Week 0 | 94.4 | 3.0 | | | Number of respondents who improved | 9 |
| Week 4 | 85.1 | 2.5 | -9.9 | 0.027182 | Proportion of respondents who improved (%) | 100 |
| **Cholesterol** | **Mean** | **Standard deviation** | **%** | **p value** | **Total number of respondents** | **9** |
| Week 0 | 206.6 | 11.0 | | | Number of respondents who improved | 7 |
| Week 4 | 198 | 8.8 | -4.2 | 0.272127 | Proportion of respondents who improved (%) | 77.8 |
| **HDL** | **Mean** | **Standard deviation** | **%** | **p value** | **Total number of respondents** | **9** |
| Week 0 | 53.85556 | 3.4 | | | Number of respondents who improved | 8 |
| Week 4 | 55.97778 | 3.5 | -3.9 | 0.0055 | Proportion of respondents who improved | 88.9 |
| **LDL** | **Mean** | **Standard deviation** | **%** | **p value** | **Total number of respondents** | **9** |
| Week 0 | 138.9 | 9.7 | | | Number of respondents who improved | 7 |
| Week 4 | 127.6 | 9.6 | -8.2 | 0.15 | Proportion of respondents who improved (%) | 77.8 |
| **LDL/HDL** | **Mean** | **Standard deviation** | **%** | **p value** | **Total number of respondents** | **9** |
| Week 0 | 2.6 | 0.3 | | | Number of respondents who improved | 7 |
| Week 4 | 2.4 | 0.3 | -9.3 | 0.101 | Proportion of respondents who improved (%) | 77.8 |

Based on FIG. 13 and Table 18, it is known that after continuously taking *Bifidobacterium breve* BCRC 911145 viable bacteria capsules for 4 weeks, total cholesterol can be lowered by 4.2% and the proportion of respondents who improved reaches 77.8%.

Furthermore, according to FIGS. 14A to 14C and Table 18, it is known that after continuously taking *Bifidobacterium breve* BCRC 911145 viable bacteria capsules for 4 weeks, high-density lipoprotein cholesterol (HDL-C) can be significantly increased by 3.9% and low-density lipoprotein cholesterol (LDL-C) can be reduced by 8.2%, while reducing the ratio of low-density lipoprotein cholesterol to high-density lipoprotein cholesterol (LDL/HDL) by about 9.3%. For the foregoing three indexes, the proportions of respondents who improved are 88.9%, 77.8% and 77.8%, respectively.

In addition, according to FIG. 15 and Table 18, it is known that after continuously taking *Bifidobacterium breve* BCRC 911145 viable bacteria capsules for 4 weeks, fasting blood glucose can be significantly reduced by 9.1%, and the proportion of respondents who improved reaches 90.0%.

From the above, it is known that *Bifidobacterium breve* BCRC 911145 viable bacteria does have the effects of reducing appetite, reducing total cholesterol and low-density lipoprotein cholesterol (LDL-C), increasing high-density lipoprotein cholesterol (HDL-C) and reducing fasting blood sugar on human body.

### D-2. Efficacy test of viable bacteria composition

### D-2-1. Efficacy test of composition containing viable bacteria and soybean extract

Aims of the experiment: To test the weight loss effect of a composition containing *Bifidobacterium breve* BCRC 911145 viable bacteria and soybean extract.

Experimental content:

1. Dosage form: powder pack
2. Dosage: 4000 mg/package of a composition containing *Bifidobacterium breve* BCRC 911145 viable bacteria and soybean extract (containing 150 mg (equivalent to $2 \times 10^8$ CFU/g) of *Bifidobacterium breve* BCRC 911145 viable bacteria powder and 150 mg of soybean extract and additives)
3. Experimental Design: Subjects took 1 pack 1 hour before lunch daily for taking 60 consecutive days.
4. Number of persons tested: 30 persons
5. Conditions for inclusion of subjects: Male or female aged 20-40
6. Test items:
Body weight and body fat

The results are shown in Table 19, and FIG. 16A and FIG. 16B.

Table 19: Changes in body weight and body fat percentage of subjects

| Subject number | Day 0 body weight (Initial body weight) | Day 0 body fat percentage (Initial body fat percentage) | Day 14 body weight | Day 14 body fat percentage | Day 28 body weight | Day 28 body fat percentage |
|---|---|---|---|---|---|---|
| 1 | 55.7 | 26.3 | 54.6 | 26.8 | 54.6 | 25.6 |
| 2 | 58 | 29.6 | 55.4 | 27 | 55 | 26.7 |
| 3 | 73.6 | 26 | 71.6 | 25.1 | 70.5 | 24.9 |
| 4 | 79 | 40.3 | 76.1 | 40.6 | 74.4 | 37.8 |
| 5 | 97.7 | 35.6 | 95.2 | 35.5 | 94.1 | 38.3 |
| 6 | 61.9 | 29.4 | 59.5 | 28.4 | 58.9 | 28 |
| 7 | 71.8 | 23.4 | 71.7 | 23.9 | 71 | 23 |
| 8 | 70.4 | 41.7 | 68.4 | 40.4 | 67.9 | 41 |
| 9 | 69 | 24.1 | 67.7 | 23.9 | 67.6 | 24.2 |
| 10 | 71.2 | 36.8 | 69.4 | 35.7 | 68.4 | 34.9 |
| 11 | 62 | 36.7 | 60.7 | 36 | 60.3 | 38.7 |
| 12 | 50 | 41.2 | 48.2 | 41.6 | 48.2 | 40.5 |
| 13 | 88.4 | 32 | 85 | 32 | 83.6 | 31.2 |
| 14 | 84.4 | 31.9 | 81.4 | 30.8 | 79.3 | 30.3 |
| 15 | 84.4 | 30.5 | 83 | 29.9 | 82.4 | 27.5 |
| 16 | 118.6 | 32.9 | 115.2 | 32.9 | 115 | 32.8 |

(continued)

| Subject number | Day 0 body weight (Initial body weight) | Day 0 body fat percentage (Initial body fat percentage) | Day 14 body weight | Day 14 body fat percentage | Day 28 body weight | Day 28 body fat percentage |
|---|---|---|---|---|---|---|
| 17 | 78.3 | 42.4 | 78 | 42 | 76 | 40 |
| 18 | 75.4 | 38.8 | 72.3 | 36.8 | 72.2 | 36.6 |
| 19 | 64 | 32.3 | 60.4 | 30.1 | 60.4 | 31.1 |
| 20 | 102.9 | 31.8 | 99.9 | 30.8 | 99.9 | 30.9 |
| 21 | 61.8 | 38.1 | 60 | 37 | 60 | 35 |
| 22 | 111.5 | 42.6 | 106.7 | 41.7 | 104.1 | 40.8 |
| 23 | 53.3 | 28.8 | 51 | 27.9 | 51 | 28 |
| 24 | 84.3 | 33.6 | 83 | 33 | 82 | 32.5 |
| 25 | 81.1 | 24.9 | 77.2 | 24.3 | 75.4 | 23.1 |
| 26 | 54.1 | 29.1 | 52.6 | 28.7 | 50.8 | 27 |
| 27 | 53.2 | 28.6 | 50.2 | 27 | 49.3 | 25.8 |
| 28 | 79.8 | 29.5 | 76 | 27 | 75 | 26.3 |
| 29 | 54.2 | 30.7 | 51.6 | 30.1 | 51.3 | 29.4 |
| 30 | 78.7 | 42.3 | 77.8 | 41.9 | 77.8 | 41.9 |

Based on Table 19, and FIG. 16A and FIG. 16B, it is known that the composition containing *Bifidobacterium breve* BCRC 911145 viable bacteria and soybean extract can indeed reduce body weight and body fat percentage, and has a weight loss effect.

**D-2-2. Efficacy test of composition containing viable bacteria, soybean extract and apple extract**

**D-2-2-1. Blood glucose modulating efficacy test of composition containing viable bacteria, soybean extract and apple extract**

**Mode 1**

Aims of the experiment: To test the blood glucose modulating effect of a composition containing *Bifidobacterium breve* BCRC 911145 viable bacteria, soybean extract and apple extract.

Experimental content:

1. Dosage form: powder pack
2. Dosage: 800 mg/package of a composition containing *Bifidobacterium breve* BCRC 911145 viable bacteria, soybean extract and apple extract (containing 150 mg (equivalent to $5 \times 10^9$ CFU/g) of *Bifidobacterium breve* BCRC 911145 viable bacteria powder, 150 mg of soybean extract, 150 mg of apple extract and additives)
3. Number of persons tested: 5 persons
4. Conditions for inclusion of subjects: Male or female aged 20-40
5. Experimental Design:
Subject self-control experiment. For the same group of subjects, blood samples were collected at the same time intervals when the subjects did not take the composition of the present disclosure (the control group) and when the subjects took the composition of the present disclosure (the group taking the composition of the present disclosure).

The subjects took 1 pack of the above composition on Day 1 of the experiment, and did not take the composition on Day 7 of the experiment. On Day 1 of the experiment, the subjects' blood was collected when they were fasting, and then the subjects were asked to take 1 pack of the above composition, and the blood of the subjects was collected 15 minutes, 45 minutes, 60 minutes, 120 minutes and 240 minutes after taking it. After 7 days of washout, the subjects' blood was collected again when they were fasting (on Day 9 of the experiment), and then the blood of the subjects was collected at 15 minutes, 45 minutes, 60 minutes, 120 minutes and 240 minutes.

6. Test items:

(1) Changes in serum GLP-1 concentration;
(2) Changes in blood glucose concentration

The results are shown in FIG. 17 and FIG. 18.

FIG. 17 shows that 60 minutes after taking the aforementioned composition, the concentration of glucagon-like peptide-1 (GLP-1) in the serum of the subjects increases significantly by 17.6%, indicating that the aforementioned composition can effectively promote the secretion of glucagon-like peptide-1 (GLP-1) in the body. In contrast, when the aforementioned composition is not taken, the concentration of glucagon-like peptide-1 (GLP-1) in the serum of the subjects fluctuated greatly. There is no trend of stable increase.

FIG. 18 shows that compared to not taking the aforementioned composition, the blood glucose level of the subjects after taking the aforementioned composition is more stable without fluctuation, indicating that taking the aforementioned composition can effectively help modulate blood glucose and achieve the effect of controlling appetite by stabilizing blood glucose.

From the above, it is known that the above composition does have the effect of modulating blood glucose and can control appetite.

**Mode 2**

Aims of the experiment: To test the blood glucose modulating effect of a composition containing *Bifidobacterium breve* BCRC 911145 viable bacteria, soybean extract and apple extract.

Experimental content:

1. Dosage form: powder pack
2. Dosage: 800 mg/package of a composition containing Bifidobacterium breve BCRC 911145 viable bacteria, soybean extract and apple extract (containing 150 mg (equivalent to $5 \times 10^9$ CFU/g) of Bifidobacterium breve BCRC 911145 viable bacteria powder, 150 mg of soybean extract, 150 mg of apple extract and additives)
3. Number of persons tested: 4 persons
4. Conditions for inclusion of subjects: Male or female aged 20-40
5. Experimental Design:
Subject self-control experiment. For the same group of subjects, blood samples were collected at the same time intervals when the subjects did not take the composition of the present disclosure (the control group) and when the subjects took the composition of the present disclosure (the group taking the composition of the present disclosure).

On Day 1 of the experiment, the subjects' blood was collected when they were fasting, and then the subjects were asked to finish breakfast (a corn Chinese omelet) within 15 minutes, and the subjects' blood was collected 2 hours and 4 hours after having breakfast. After an interval of 3 days (the Day 5 of the experiment), the subjects' blood was collected when they were fasting, and then the subjects were asked to take 1 pack of the above-mentioned composition and finish the same breakfast as before within 15 minutes, and the subjects' blood was collected 2 hours and 4 hours after having breakfast. After the subjects took the above-mentioned composition for 3 consecutive days (Day 5 to Day 7 of the experiment), the subjects' blood was collected again when they were fasting (Day 8 of the experiment).

6. Test items:

(1) Changes in serum glucagon-like peptide-1 (GLP-1) concentration;

(2) Changes in blood glucose concentration and changes in insulin concentration;
(3) Fasting blood glucose concentration;
(4) Appetite survey.

The results are shown in FIG. 19, FIG. 20A and FIG. 20B, FIG. 21, and FIG. 22A to FIG. 22C.

FIG. 19 shows that after 2 hours and 4 hours of taking the above composition, the serum glucagon-like peptide-1 (GLP-1) of the subjects continue to increase steadily, which means that taking the above composition can effectively promote the secretion of glucagon-like peptide-1 (GLP-1) in the body and help modulate blood glucose and control appetite. In contrast, the serum glucagon-like peptide-1 (GLP-1) concentration of the control group does not increase.

According to FIG. 20A and FIG. 20B, it is known that 4 hours after having breakfast, the subjects' postprandial blood glucose increases significantly. In contrast, 4 hours after having the above composition, the increase level in postprandial blood glucose of the subjects is significantly lower than that of the control group, which indicates that the above composition can effectively help stabilize postprandial blood glucose.

Based on FIG. 21, it is known that after taking the aforementioned composition for 3 consecutive days, the fasting blood glucose of the subjects improves, which means that the aforementioned composition can effectively help stabilize fasting blood glucose.

Furthermore, based on FIG. 22A to FIG. 22C, it is known that after taking the above composition, the subjects' feeling of hunger and appetite level 4 hours after finishing breakfast both are lower than those of the control group. The subjects are less likely to feel hungry and less likely to experience sporadic food cravings before lunch.

From the above, it is known that the above composition does have the effects of modulating blood glucose and reducing appetite.

Although the present disclosure has been disclosed as above with preferred embodiments, it is not intended to limit the present disclosure. Anyone skilled in the art may make some changes and modifications without departing from the spirit and scope of the present disclosure. Therefore, the protection scope of the present disclosure shall be subject to the scope of the claims attached hereto.

Deposit of biological material

**Domestic deposit**

  *1. Bifidobacterium breve, Bifidobacterium breve* TCI761

  Bioresource Collection and Research Center of Food Industry Research and Development Institute, Taiwan, China
  August 8, 2022
  BCRC 911145

**Overseas deposit**

  *1. Bifidobacterium breve, Bifidobacterium breve* TCI761

  German Collection of Microorganisms and Cell Cultures GmbH (DSMZ)
  September 7, 2022
  DSM 34383

**Claims**

**1.** A novel *Bifidobacterium breve* which is deposited in the Bioresource Collection and Research Center of Food Industry Research and Development Institute, and the deposit number of which is BCRC 911145.

**2.** Use of the culture as claimed in claim 1 in the manufacture of a composition for reducing appetite and/or promoting glucagon-like peptide-1 (GLP-1) secretion.

3. Use of the novel *Bifidobacterium breve* as claimed in claim 1 or a culture thereof in the manufacture of a composition for inhibiting adipocyte maturation.

4. Use of the novel *Bifidobacterium breve* as claimed in claim 1 or a culture thereof in the manufacture of a composition for reducing fat accumulation and/or promoting fat metabolism.

5. Use of the novel *Bifidobacterium breve* as claimed in claim 1 or a culture thereof in the manufacture of a composition for reducing body weight.

6. Use of the novel *Bifidobacterium breve* as claimed in claim 1 or a culture thereof in the manufacture of a composition for modulating blood lipids.

7. The use as claimed in claim 6, wherein the modulating blood lipids comprise:

reducing total cholesterol;
promoting high-density lipoprotein cholesterol (HDL-C) synthesis; and/or
promoting low-density lipoprotein cholesterol (LDL-C) metabolism.

8. Use of the novel *Bifidobacterium breve* as claimed in claim 1 or a culture thereof in the manufacture of a composition for modulating blood glucose.

9. The use as claimed in any one of claims 2, 3, 4, 5, 6 and 8, wherein the culture comprises the novel *Bifidobacterium breve* which has been cultured.

10. The use as claimed in any one of claims 2, 3, 4, 5, 6 and 8, wherein the novel *Bifidobacterium breve* which has been cultured is active or a viable bacterium.

11. The use as claimed in any one of claims 2, 3, 4, 5, 6 and 8, wherein the novel *Bifidobacterium breve* which has been cultured has been stripped of its own physiological activity or is a dead bacterium.

12. The use as claimed in any one of claims 2, 3, 4, 5, 6 and 8, wherein the culture comprises a cultured supernatant of the novel *Bifidobacterium breve.*

13. A probiotic composition, comprising:

the novel *Bifidobacterium breve* as claimed in claim 1 or a culture thereof; and
a prebiotic.

14. The probiotic composition as claimed in claim 13, wherein the weight ratio of the novel *Bifidobacterium breve* or the culture thereof to the prebiotic is 1-6:1-12.

15. The probiotic composition as claimed in claim 13, wherein the culture comprises the novel *Bifidobacterium breve* which has been cultured.

16. The probiotic composition as claimed in claim 15, wherein the novel *Bifidobacterium breve* which has been cultured is active or a viable bacterium.

17. The probiotic composition as claimed in claim 15, wherein the novel *Bifidobacterium breve* which has been cultured has been stripped of its own physiological activity or is a dead bacterium.

18. The probiotic composition as claimed in claim 13, wherein the culture comprises a cultured supernatant of the novel *Bifidobacterium breve.*

19. The probiotic composition as claimed in claim 13, wherein the prebiotic comprises at least one of the following ingredients:
a soybean extract, an oat extract, a barley extract, an apple extract, a banana extract, a tomato extract, a chicory extract, an asparagus extract, a beet extract, a garlic extract, an onion extract, a seaweed extract, a pectin extract and a tea extract.

**20.** The probiotic composition as claimed in claim 19, wherein the prebiotic comprises a soybean extract.

**21.** The probiotic composition as claimed in claim 19, wherein the prebiotic comprises a soybean extract and an apple extract.

**22.** The probiotic composition as claimed in claim 13, wherein the probiotic composition is a food composition.

FIG. 1A

FIG. 1B

GLP-1

FIG. 2A

PYY

FIG. 2B

C/EBPA

FIG. 3A

Cultured supernatant of *Bifidobacterium breve* (the final concentration in the medium for culturing the cells is 0.25%)

Fat accumulation

FIG. 3B

Cultured supernatant of *Bifidobacterium breve* (the final concentration in the medium for culturing the cells is 0.25%)

FIG. 4A

FIG. 4B

FIG. 5

FIG. 6A

PLIN1

▼82.3%

***

Mock | Cultured supernatant of *Bifidobacterium breve* (the final concentration in the medium for culturing the cells is 0.25%)

FIG. 6B

PPARG2

▼81.2%

***

Mock | Cultured supernatant of *Bifidobacterium breve* (the final concentration in the medium for culturing the cells is 0.125%)

FIG. 6C

LIPE(HSL)

▲3.39X

FIG. 6D

ATGL

▲16.8%

FIG. 7A

PLIN1

FIG. 7B

PPARG2

FIG. 7C

FIG. 7D

FIG. 8A

FIG. 8B

FIG. 8C

FIG. 8D

FIG. 9

FIG. 10

FIG. 11

FIG. 12

FIG. 13

FIG. 14A

FIG. 14B

LDL/HD Lratio

FIG. 14C

FIG. 15

FIG. 16A

FIG. 16B

FIG. 17

FIG. 18

FIG. 19

FIG. 20A

FIG. 20B

FIG. 21

FIG. 22A

FIG. 22B

Compared to normal conditions, overall appetite changes after taking the composition of the present disclosure

FIG. 22C

## INTERNATIONAL SEARCH REPORT

| International application No. |
|---|
| **PCT/CN2024/073257** |

**A. CLASSIFICATION OF SUBJECT MATTER**

C12N 1/20(2006.01)i; A61K 35/745(2015.01)i; A61P 3/04(2006.01)i; A61P 3/06(2006.01)i; C12R 1/225(2006.01)n

According to International Patent Classification (IPC) or to both national classification and IPC

**B. FIELDS SEARCHED**

Minimum documentation searched (classification system followed by classification symbols)

IPC:C12N,A61K,A61P,C12R

Documentation searched other than minimum documentation to the extent that such documents are included in the fields searched

Electronic data base consulted during the international search (name of data base and, where practicable, search terms used)

CNTXT, ENTXTC, VEN, ENTXT, CNKI, 百度学术, BAIDU SCHOLAR, PubMed, ISI-WEB OF SCIENCE: 短双歧杆菌, bifidobacterium breve, 类升糖素胜肽, 胰高(血)糖素样肽, glucagon-like peptide, 脂肪, fat, 体重, weight, 胆固醇, cholesterol, cholesterin, 高密度脂蛋白, high density lipoprotein, 低密度脂蛋白, low density lipoprotein

**C. DOCUMENTS CONSIDERED TO BE RELEVANT**

| Category* | Citation of document, with indication, where appropriate, of the relevant passages | Relevant to claim No. |
|---|---|---|
| A | WO 2022005035 A1 (IL DONG PHARMACEUTICAL CO., LTD.) 06 January 2022 (2022-01-06)<br>see abstract, and claims 1-14 | 1-22 |
| A | CN 111996153 A (NANJING YIRUILAN BIOLOGICAL TECHNOLOGY CO., LTD.) 27 November 2020 (2020-11-27)<br>see description, paragraphs [0006]-[0014] | 1-22 |
| A | US 2017333495 A1 (BIOSEARCH, S.A.) 23 November 2017 (2017-11-23)<br>see description, paragraphs [0006]-[0020] | 1-22 |
| A | 余仁强 等人 (YU, Renqiang et al.). "益生菌对肥胖大鼠血脂紊乱及胰岛素抵抗的影响 (Probiotics Improve Obesity-associated Dyslipidemia and Insulin Resistance in High-fat Diet-fed Rats)"<br>中国当代儿科杂志 (Chinese Journal of Contemporary Pediatrics),<br>Vol. 15, No. 12, 19 December 2013 (2013-12-19),<br>see pages 1123-1127 | 1-22 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **23 April 2024** | **30 April 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/073257** |

| C. | DOCUMENTS CONSIDERED TO BE RELEVANT | |
| --- | --- | --- |
| Category* | Citation of document, with indication, where appropriate, of the relevant passages | Relevant to claim No. |
| A | Hyun Kyung Sung et al. "Body Fat Reduction Effect of Bifidobacterium breve B-3: A Randomized, Double-Blind, Placebo Comparative Clinical Trial" *Nutrients*, Vol. 15, No. 1, 21 December 2022 (2022-12-21), see pages 1-11 | 1-22 |

Form PCT/ISA/210 (second sheet) (July 2022)

## INTERNATIONAL SEARCH REPORT
### Information on patent family members

International application No.

**PCT/CN2024/073257**

| Patent document cited in search report | | | Publication date (day/month/year) | Patent family member(s) | | | Publication date (day/month/year) |
|---|---|---|---|---|---|---|---|
| WO | 2022005035 | A1 | 06 January 2022 | CA | 3184231 | A1 | 06 January 2022 |
| | | | | AU | 2021299360 | A1 | 16 February 2023 |
| | | | | US | 2023285474 | A1 | 14 September 2023 |
| | | | | KR | 102301647 | B1 | 13 September 2021 |
| | | | | KR | 102256507 | B1 | 26 May 2021 |
| | | | | JP | 2023531714 | A | 25 July 2023 |
| | | | | TW | 202207957 | A | 01 March 2022 |
| CN | 111996153 | A | 27 November 2020 | None | | | |
| US | 2017333495 | A1 | 23 November 2017 | EP | 3230479 | A1 | 18 October 2017 |
| | | | | EP | 3230479 | B1 | 06 October 2021 |
| | | | | ES | 2899185 | T3 | 10 March 2022 |
| | | | | ES | 2899185 | T8 | 26 May 2022 |
| | | | | JP | 2018503367 | A | 08 February 2018 |
| | | | | JP | 6707087 | B2 | 10 June 2020 |
| | | | | BR | 112017012251 | A2 | 30 January 2018 |
| | | | | HUE | 057081 | T2 | 28 April 2022 |
| | | | | PL | 3230479 | T3 | 09 May 2022 |
| | | | | AU | 2015359382 | A1 | 27 July 2017 |
| | | | | AU | 2015359382 | B2 | 02 September 2021 |
| | | | | MX | 2017007654 | A | 04 May 2018 |
| | | | | EP | 3031930 | A1 | 15 June 2016 |
| | | | | RU | 2017121227 | A | 10 January 2019 |
| | | | | RU | 2017121227 | A3 | 28 May 2019 |
| | | | | RU | 2722038 | C2 | 26 May 2020 |
| | | | | CA | 2970602 | A1 | 16 June 2016 |
| | | | | CA | 2970602 | C | 12 March 2024 |
| | | | | WO | 2016092032 | A1 | 16 June 2016 |
| | | | | RS | 62610 | B1 | 31 December 2021 |
| | | | | US | 11058734 | B2 | 13 July 2021 |

Form PCT/ISA/210 (patent family annex) (July 2022)